# EUROPÄISCHE PATENTSCHRIFT

(11) **EP 2 665 826 B1**
(45) Veröffentlichungstag und Bekanntmachung des Hinweises auf die Patenterteilung: **01.11.2017**
(21) Anmeldenummer: 12700132.9
(22) Anmeldetag: 12.01.2012
(51) Int. Cl.: C12P 13/12, C12N 1/20

(54) **VERFAHREN ZUR FERMENTATIVEN HERSTELLUNG SCHWEFELHALTIGER AMINOSÄUREN**
PROCESS FOR THE FERMENTATIVE PRODUCTION OF SULPHUR-CONTAINING AMINO ACIDS
PROCÉDÉ DE FABRICATION FERMENTATIVE D'ACIDES AMINÉS À TENEUR EN SOUFRE

(30) Priorität: 20.01.2011 EP 11151526
(43) Veröffentlichungstag der Anmeldung: 27.11.2013
(73) Patentinhaber: Evonik Degussa GmbH, 45128 Essen (DE)
(72) Erfinder: SCHNEIDER, Frank, 33790 Halle (DE); MOLCK, Stella, 33602 Bielefeld (DE); BATHE, Brigitte, 33154 Salzkotten (DE)
(86) Internationale Anmeldenummer: PCT/EP2012/050417
(87) Internationale Veröffentlichungsnummer: WO 2012/098042

(56) Entgegenhaltungen:
- EP-A1- 1 528 108
- WO-A1-01/27307
- WO-A1-2007/077041
- WO-A2-2007/011939
- WO-A2-2007/135188
- WO-A2-2009/043803
- KRÖMER J O ET AL: "Metabolic pathway analysis for rational design of L-methionine production by Escherichia coli and Corynebacterium glutamicum", METABOLIC ENGINEERING, ACADEMIC PRESS, US, Bd. 8, Nr. 4, 1. Juli 2006 (2006-07-01), Seiten 353-369, XP024946939, ISSN: 1096-7176, DOI: 10.1016/J.YMBEN.2006.02.001 [gefunden am 2006-07-01] in der Anmeldung erwähnt
- DOROTHEA KESSLER: "Enzymatic activation of sulfur for incorporation into biomolecules in prokaryotes", FEMS MICROBIOLOGY REVIEWS, Bd. 30, Nr. 6, 1. November 2006 (2006-11-01), Seiten 825-840, XP055010872, ISSN: 0168-6445, DOI: 10.1111/j.1574-6976.2006.00036.x
- NURIA NÁRDIZ ET AL: "A rhodanese-like protein is highly overrepresented in the mutant S. clavuligerus oppA2::aph: effect on holomycin and other secondary metabolites production", MICROBIAL BIOTECHNOLOGY, Bd. 4, Nr. 2, 2. November 2010 (2010-11-02), Seiten 216-225, XP055010806, ISSN: 1751-7915, DOI: 10.1111/j.1751-7915.2010.00222.x
- HUI CHENG ET AL: "Biochemical and Genetic Characterization of PspE and GlpE, Two Singledomain Sulfurtransferases of Escherichia coli", THE OPEN MICROBIOLOGY JOURNAL, Bd. 2, Nr. 1, 25. März 2008 (2008-03-25), Seiten 18-28, XP055010808, ISSN: 1874-2858, DOI: 10.2174/1874285800802010018
- M. W. FOSTER ET AL: "A protein microarray-based analysis of S-nitrosylation", PROCEEDINGS OF THE NATIONAL ACADEMY OF SCIENCES, Bd. 106, Nr. 45, 10. November 2009 (2009-11-10), Seiten 18948-18953, XP055011290, ISSN: 0027-8424, DOI: 10.1073/pnas.0900729106

### SEQUENCE LISTING

<110> Evonik Degussa GmbH
<120> Verfahren zur fermentativen Herstellung schwefelhaltiger Aminosäuren
<130> 201000424
<160> 14
<170> PatentIn version 3.3
<210> 1
   <211> 450
   <212> DNA
   <213> Saccharomyces cerevisiae
<400> 1
   atgttcaagc atagtacagg tattctctcg aggacagttt ctgcaagatc gcctacattg      60
   gtcctgagaa catttacaac gaaggctcca aagatctata cttttgacca ggtcaggaac     120
   ctagtcgaac accccaatga taaaaaacta ttggtagatg taagggaacc caaggaagta     180
   aaggattaca agatgccaac tacaataaat attccggtga atagtgcccc tggcgctctt     240
   ggattgcccg aaaaggagtt tcacaaagtt ttccaatttg ctaaaccacc tcacgataaa     300
   gaattgattt ttctttgtgc gaaaggagta agagccaaaa ctgccgaaga gttggctcga     360
   tcttatgggt acgaaaacac tggtatctat cctggttcta ttactgagtg gttagctaaa     420
   ggtggtgctg acgttaagcc caaaaaataa                                      450
**<210>** 2
   <211> 149
   <212> PRT
   <213> Saccharomyces cerevisiae
<400> 2
   Met Phe Lys His Ser Thr Gly Ile Leu Ser Arg Thr Val Ser Ala Arg
   1               5                   10                  15
   Ser Pro Thr Leu Val Leu Arg Thr Phe Thr Thr Lys Ala Pro Lys Ile
               20                  25                  30
   Tyr Thr Phe Asp Gln Val Arg Asn Leu Val Glu His Pro Asn Asp Lys
           35                  40                  45
   Lys Leu Leu Val Asp Val Arg Glu Pro Lys Glu Val Lys Asp Tyr Lys
       50                  55                  60
   Met Pro Thr Thr Ile Asn Ile Pro Val Asn Ser Ala Pro Gly Ala Leu
   65                  70                  75                  80
   Gly Leu Pro Glu Lys Glu Phe His Lys Val Phe Gln Phe Ala Lys Pro
                   85                  90                  95
   Pro His Asp Lys Glu Leu Ile Phe Leu Cys Ala Lys Gly Val Arg Ala
               100                 105                 110
   Lys Thr Ala Glu Glu Leu Ala Arg Ser Tyr Gly Tyr Glu Asn Thr Gly
           115                 120                 125
   Ile Tyr Pro Gly Ser Ile Thr Glu Trp Leu Ala Lys Gly Gly Ala Asp
       130                 135                 140
   Val Lys Pro Lys Lys
   145
<210> 3
   <211> 450
   <212> DNA
   <213> Saccharomyces cerevisiae
<400> 3
   atgttcaaac acagcaccgg tattctgtct cgtacagttt cagcacgcag tccgacttta      60
   gtgcttcgga cctttacaac gaaagcccct aagatctata cttttgatca ggtccgaaat     120
   ctggtagaac atccaaacga caaaaagttg ctcgttgatg tgagagaacc caaagaggtc     180
   aaagattaca aaatgccgac cacgataaat attccagtta actccgctcc gggagcgctg     240
   ggcctacctg aaaaagagtt tcataaggta tttcagttcg caaaaccgcc tcatgacaaa     300
   gaactgatct tcttatgtgc caagggggtg cgtgcgaaaa cagcagagga acttgctcgc     360
   tcgtatggtt atgaaaatac aggtatttac ccaggcagca taactgagtg gctggccaaa     420
   gggggagcgg atgttaaacc caaaaagtaa                                       450
<210> 4
   <211> 450
   <212> DNA
   <213> Saccharomyces cerevisiae
<400> 4
   atgtttaaac attccactgg cattttgtcc cgtaccgtat cagcccgttc tccgaccctt 60
   gtcctgcgta ccttcactac caaagctccc aaaatctata cctttgacca ggtgcgcaac 120
   ctggttgaac atccgaacga taaaaaactg ctcgtagacg tccgtgaacc gaaagaagtg 180
   aaagactaca aaatgccaac tactattaat atcccggtta acagcgcgcc gggtgctctt 240
   ggtctgccgg aaaaagaatt ccacaaagta ttccagttcg ctaaaccgcc gcacgacaaa 300
   gaactgattt tcttatgcgc gaagggtgtg cgtgctaaaa ccgcggaaga actggctcgt 360
   tcttacggtt acgaaaacac cggcatttat ccgggctcta tcaccgaatg gctggcgaaa 420
   ggtggtgctg atgtgaagcc gaagaaataa 450
<210> 5
   <211> 450
   <212> DNA
   <213> Saccharomyces cerevisiae
<400> 5
   atgttcaaac actctaccgg tatcctgtct cgtaccgttt ctgcgcgttc tccgaccctg 60
   gttctgcgta ccttcaccac caaagcgccg aaaatctaca ccttcgacca ggttcgtaac 120
   ctggttgaac acccgaacga caaaaaactg ctggttgacg ttcgtgaacc gaaagaagtt 180
   aaagactaca aaatgccgac caccatcaac atcccggtta actctgcgcc gggtgcgctg 240
   ggtctgccgg aaaaagaatt ccacaaagtt ttccagttcg cgaaaccgcc gcacgacaaa 300
   gaactgatct tcctgtgcgc gaaaggtgtt cgtgcgaaaa ccgcggaaga actggcgcgt 360
   tcttacggtt acgaaaacac cggtatctac ccgggttcta tcaccgaatg gctggcgaaa 420
   ggtggtgcgg acgttaaacc gaaaaaataa 450
<210> 6
   <211> 58
   <212> DNA
   <213> Synthetic Sequence
<400> 6
   ggtaccttaa ttaagtggat tcgaggccgg ccacgagtac tattaaagag gagaaata 58
<210> 7
   <211> 144
   <212> DNA
   <213> Synthetic Sequence
<400> 7
   taaccggctt atcggtcagt ttcacctgat ttacgtaaaa acccgcttcg gcgggttttt     60
   gcttttggag gggcagaaag atgaatgact gtccacgacg ctatacccaa aagaaagttt    120
   aaacgcatac cctgcaggga gctc                                           144
<210> 8
   <211> 3010
   <212> DNA
   <213> Synthetic Sequence
<400> 8
   ctaaattgta agcgttaata ttttgttaaa attcgcgtta aatttttgtt aaatcagctc     60
   attttttaac caataggccg aaatcggcaa aatcccttat aaatcaaaag aatagaccga    120
   gatagggttg agtggccgct acagggcgct cccattcgcc attcaggctg cgcaactgtt    180
   gggaagggcg tttcggtgcg ggcctcttcg ctattacgcc agctggcgaa agggggatgt    240
   gctgcaaggc gattaagttg ggtaacgcca gggttttccc agtcacgacg ttgtaaaacg    300
   acggccagtg agcgcgacgt aatacgactc actatagggc gaattggcgg aaggccgtca    360
   aggcctaggc gcgccatgag ctccctgcag ggtatgcgtt taaactttct tttgggtata    420
   gcgtcgtgga cagtcattca tctttctgcc cctccaaaag caaaaacccg ccgaagcggg    480
   tttttacgta aatcaggtga aactgaccga taagccggtt attatttttt gggcttaacg    540
   tcagcaccac ctttagctaa ccactcagta atagaaccag gatagatacc agtgttttcg    600
   tacccataag atcgagccaa ctcttcggca gttttggctc ttactccttt cgcacaaaga    660
   aaaatcaatt ctttatcgtg aggtggttta gcaaattgga aaactttgtg aaactccttt    720
   tcgggcaatc caagagcgcc aggggcacta ttcaccggaa tatttattgt agttggcatc    780
   ttgtaatcct ttacttcctt gggttccctt acatctacca atagtttttt atcattgggg    840
   tgttcgacta ggttcctgac ctggtcaaaa gtatagatct ttggagcctt cgttgtaaat    900
   gttctcagga ccaatgtagg cgatcttgca gaaactgtcc tcgagagaat acctgtacta    960
   tgcttgaaca ttatttctcc tctttaatag tactcgtggc cggcctcgaa tccacttaat   1020
   taaggtacct cttaattaac tggcctcatg ggccttccgc tcactgcccg ctttccagtc   1080
   gggaaacctg tcgtgccagc tgcattaaca tggtcatagc tgtttccttg cgtattgggc   1140
   gctctccgct tcctcgctca ctgactcgct gcgctcggtc gttcgggtaa agcctggggt   1200
   gcctaatgag caaaaggcca gcaaaaggcc aggaaccgta aaaaggccgc gttgctggcg   1260
   tttttccata ggctccgccc ccctgacgag catcacaaaa atcgacgctc aagtcagagg   1320
   tggcgaaacc cgacaggact ataaagatac caggcgtttc cccctggaag ctccctcgtg   1380
   cgctctcctg ttccgaccct gccgcttacc ggatacctgt ccgcctttct cccttcggga   1440
   agcgtggcgc tttctcatag ctcacgctgt aggtatctca gttcggtgta ggtcgttcgc   1500
   tccaagctgg gctgtgtgca cgaacccccc gttcagcccg accgctgcgc cttatccggt   1560
   aactatcgtc ttgagtccaa cccggtaaga cacgacttat cgccactggc agcagccact   1620
   ggtaacagga ttagcagagc gaggtatgta ggcggtgcta cagagttctt gaagtggtgg   1680
   cctaactacg gctacactag aagaacagta tttggtatct gcgctctgct gaagccagtt   1740
   accttcggaa aaagagttgg tagctcttga tccggcaaac aaaccaccgc tggtagcggt   1800
   ggtttttttg tttgcaagca gcagattacg cgcagaaaaa aaggatctca agaagatcct   1860
   ttgatctttt ctacggggtc tgacgctcag tggaacgaaa actcacgtta agggattttg   1920
   gtcatgagat tatcaaaaag gatcttcacc tagatccttt taaattaaaa atgaagtttt   1980
   aaatcaatct aaagtatata tgagtaaact tggtctgaca gttaccaatg cttaatcagt   2040
   gaggcaccta tctcagcgat ctgtctattt cgttcatcca tagttgcctg actccccgtc   2100
   gtgtagataa ctacgatacg ggagggctta ccatctggcc ccagtgctgc aatgataccg   2160
   cgagaaccac gctcaccggc tccagattta tcagcaataa accagccagc cggaagggcc 2220
   gagcgcagaa gtggtcctgc aactttatcc gcctccatcc agtctattaa ttgttgccgg 2280
   gaagctagag taagtagttc gccagttaat agtttgcgca acgttgttgc cattgctaca 2340
   ggcatcgtgg tgtcacgctc gtcgtttggt atggcttcat tcagctccgg ttcccaacga 2400
   tcaaggcgag ttacatgatc ccccatgttg tgcaaaaaag cggttagctc cttcggtcct 2460
   ccgatcgttg tcagaagtaa gttggccgca gtgttatcac tcatggttat ggcagcactg 2520
   cataattctc ttactgtcat gccatccgta agatgctttt ctgtgactgg tgagtactca 2580
   accaagtcat tctgagaata gtgtatgcgg cgaccgagtt gctcttgccc ggcgtcaata 2640
   cgggataata ccgcgccaca tagcagaact ttaaaagtgc tcatcattgg aaaacgttct 2700
   tcggggcgaa aactctcaag gatcttaccg ctgttgagat ccagttcgat gtaacccact 2760
   cgtgcaccca actgatcttc agcatctttt actttcacca gcgtttctgg gtgagcaaaa 2820
   acaggaaggc aaaatgccgc aaaaaaggga ataagggcga cacggaaatg ttgaatactc 2880
   atactcttcc tttttcaata ttattgaagc atttatcagg gttattgtct catgagcgga 2940
   tacatatttg aatgtattta gaaaaataaa caaatagggg ttccgcgcac atttccccga 3000
   aaagtgccac 3010
<210> 9
   <211> 20
   <212> DNA
   <213> Synthetic Sequence
<400> 9
   tactgccgcc aggcaaattc 20
<210> 10
   <211> 20
   <212> DNA
   <213> Synthetic Sequence
<400> 10
   tttgcgccga catcataacg 20
<210> 11
   <211> 55
   <212> DNA
   <213> Synthetic Sequence
<400> 11
   aggacagtcg acggtaccgc aagcttcggc ttcgcactgg aaagcgggca gtgag 55
<210> 12
   <211> 20
   <212> DNA
   <213> Synthetic Sequence
<400> 12
   agcgcgacgt aatacgactc 20
<210> 13
   <211> 20
   <212> DNA
   <213> Synthetic Sequence
<400> 13
   atgtggaagc cggactagac 20
<210> 14
   <211> 20
   <212> DNA
   <213> Synthetic Sequence
<400> 14
   tcggattatc ccgtgacagg 20
201000424 5

## Beschreibung

Die Erfindung betrifft ein Verfahren zur fermentativen Herstellung schwefelhaltiger Aminosäuren ausgewählt aus der Gruppe L-Methionin, L-Cystein, L-Cystin, L-Homocystein und L-Homocystin.

Schwefelhaltige Aminosäuren haben eine große wirtschaftliche Bedeutung. L-Cystein wird als Lebensmittelzusatz, als Ausgangsstoff für pharmakologische Wirkstoffe (z.B. N-Acetylcystein) und für Kosmetika verwendet. Die Aminosäure L-Methionin spielt eine herausragende Rolle in der Tierernährung. Sie gehört zu den essentiellen Aminosäuren, die im Stoffwechsel der Wirbeltiere nicht durch Biosynthese hergestellt werden können. Demzufolge muss bei der Tierzucht sichergestellt werden, dass Methionin in ausreichenden Mengen mit der Nahrung aufgenommen wird. Da L-Methionin aber in klassischen Futtermittelpflanzen (wie Soja oder Getreide) insbesondere für Schweine und Geflügel häufig in zu geringen Mengen vorhanden ist, um eine optimale Tierernährung zu gewährleisten, ist es vorteilhaft, Methionin als Zusatzstoff dem Tierfutter beizumischen. D-Methionin kann von Wirbeltieren in biologisch aktives L-Methionin überführt werden. Daher wird dem Tierfutter meist ein Racemat aus D- und L-Methionin zugesetzt. L-Homocystein kann von Tieren durch Transmethylierung zu L-Methionin umgesetzt werden und dieses somit ersetzen.

Im Stand der Technik werden Aminosäuren wie Methionin durch chemische Synthese hergestellt. Dabei werden zunächst Acrolein und Methylmercaptan zu 3-Methylthiopropionaldehyd umgesetzt, der wiederum mit Cyanid, Ammoniak und Kohlenmonoxid zum Hydantoin führt. Dieses kann letztendlich zum Razemat, einem äquimolaren Gemisch der beiden Stereoisomeren D-bzw. L-Methionin hydrolysiert werden. Da die biologisch aktive Form des Moleküls ausschließlich die L-Form repräsentiert, muss die im Futter enthaltene D-Form im Stoffwechsel erst durch Des- und Transaminierung in die aktive L-Form überführt werden.

Im Gegensatz zu Methionin werden die meisten anderen natürlichen, proteinogenen Aminosäuren vorwiegend durch Fermentation von Mikroorganismen hergestellt. Dabei wird ausgenutzt, dass Mikroorganismen über entsprechende Biosynthesewege zur Synthese der natürlichen Aminosäuren verfügen. Zudem erreichen viele Fermentationsverfahren mit kostengünstigen Edukten wie Glucose und Mineralsalzen sehr günstige Herstellkosten und liefern zudem die biologisch aktive L-Form der jeweiligen Aminosäure.

Biosynthesewege von Aminosäuren unterliegen jedoch in Wildtyp-Stämmen einer strengen metabolischen Kontrolle, die gewährleistet, dass die Aminosäuren nur zum Eigenbedarf der Zelle hergestellt werden. Eine wichtige Voraussetzung für effiziente Produktionsprozesse ist es deshalb, dass geeignete Mikroorganismen verfügbar sind, die im Gegensatz zu Wildtyp-Organismen eine drastisch gesteigerte Produktionsleistung für die Herstellung der gewünschten Aminosäure aufweisen.

Solche Aminosäure-überproduzierenden Mikroorganismen können durch klassische Mutations-/Selektionsverfahren und/oder durch moderne, gezielte, rekombinante Techniken ("metabolic engineering") erzeugt werden. Bei letzterem werden zunächst Gene oder Allele identifiziert, die durch ihre Veränderung, Aktivierung oder Inaktivierung eine Aminosäure-Überproduktion bewirken. Diese Gene/Allele werden dann durch molekularbiologische Techniken in einen Mikroorganismenstamm eingebracht oder inaktiviert, so dass eine optimale Überproduktion erzielt wird. Häufig führt jedoch erst die Kombination mehrerer, verschiedener Maßnahmen zu einer wirklich effizienten Produktion.

In *E. coli* und *C. glutamicum* leitet sich L-Cystein biochemisch von L-Serin ab. L-Serin wird durch die Serin-Acetyltransferase CysE als O-Acetylserin aktiviert. Die O-Acetylserin (thiol)-Lyase überträgt anschließend reduzierten Schwefel in Form von Sulfid, wodurch L-Cystein entsteht. Im Gegensatz zu *C*. *glutamicum* besitzt *E. coli* zwei verschiedene O-Acetylserin (thiol)-Lyasen, wobei die o-Acetylserin (thiol)-Lyase B ("CysM") auch Thiosulfat auf o-Acetylserin übertragen kann. Dadurch entsteht S-Sulfocystein, welches anschließend auf bisher nicht charakterisierte Weise zu L-Cystein und Sulfit oder Sulfat gespalten wird (Kredich N.M. (1996) in Neidhardt FC et al. (Hrsg.) "Escherichia coli and Salmonella", 2. Ausgabe, S. 514-527).

L-Methionin leitet sich zusammen mit Lysin und Threonin vom Aspartat ab. Schwefel wird in Form von L-Cystein (über Cystathionin als Zwischenprodukt) durch Transsulfurierung in L-Methionin eingebracht (in *C*. *glutamicum* und *E. coli;* einziger Weg in *E. coli*). Parallel dazu gibt es z.B. bei *C. glutamicum* den Weg der direkten Sulfhydrylierung, bei dem Sulfid in Form von L-Homocystein assimiliert wird (B-J Hwang, H-J Yeom, Y Kim, H-S Lee, 2002, J Bacteriol., 184(5): 1277-1286). Die C1-Gruppe des L-Methionins stammt aus dem C1-Stoffwechsel und sie wird von den Methionin-Synthasen MetE und MetH auf L-Homocystein übertragen (Review: Greene RC (1996) in Neidhardt FC et al. (Hrsg.) "Escherichia coli and Salmonella", 2. Ausgabe, S. 542-560). Die L-Methionin-Biosynthese bei *C. glutamicum* wird bei Rückert et al. beschrieben (Rückert C, Pühler A, Kalinowski J, 2003, J Biotechnol., 104(1-3):213-28). Stämme und Verfahren zur fermentativen Produktion von L-Methionin wurden z.B. für *E. coli* (WO2006/001616), WO2009/043803) und *C. glutamicum* (WO2009/144270) beschrieben.

Die Verwendung von Thiosulfat als Schwefelquelle erlaubt bei der Produktion schwefelhaltiger Aminosäuren (gegenüber Sulfat) deutlich höhere theoretische Ausbeuten (Krömer JO, Wittmann C, Schröder H, Heinzle E, Metab Eng., 2006, 8(4), pp. 353-369; sowie WO2007/020295). Der Vorteil des Thiosulfates erklärt sich wie folgt: In Sulfat (SO₄²⁻) liegt das Schwefelatom in der Oxidationsstufe +6 vor. Für die Assimilation muss es auf die Oxidationsstufe -2 (Sulfid = S²⁻) reduziert werden. Für die Reduktion eines Sulfates zum Sulfid muss die Zelle 2 ATP und 4 NADPH (= 8 Elektronen) aufwenden. Im Thiosulfat hat das zentrale Schwefelatom die Oxidationsstufe +5 und das terminale die Oxidationsstufe -1 (durchschnittliche formale Oxidationsstufe beider Schwefelartome: +2). Zur Reduktion beider Schwefelatome des Thiosulfates werden daher nur 8 Elektronen benötigt, gegenüber 16 Elektronen bei der Reduktion von 2 Sulfaten.

Die Verwendung von Thiosulfat als S-Quelle für die Produktion von L-Cystein mit *E. coli* wird z.B. in DE 10 2007 007 333 und WO2001/27307 beschrieben.

In WO2007/077041 wurde gezeigt, das sich auch die L-Methionin-Produktion bei *E. coli* durch die Verwendung von Thiosulfat statt Sulfat signifikant verbessern lässt.

Es wurde bisher noch nicht experimentell gezeigt, dass die Verwendung von Thiosulfat auch bei *C*. *glutamicum* zur Verbesserung der L-Methioninproduktion führt. In WO2007/020295 (S. 240) wird gezeigt, dass *C*. *glutamicum* mit Na-Thiosulfat als Schwefelquelle mehr Biomasse bildet. Dies wurde als Zeichen für den geringeren ATP- und NADPH-Verbrauch gewertet. L-Methionin-Produktion mit Thiosulfat wurde nicht praktisch untersucht.

In *E. coli* wird Thiosulfat durch den Sulfat-/Thiosulfattransporter CysPUWA-Sbp aufgenommen (Kredich N.M. (1996) in Neidhardt FC et al. (Hrsg.) "Escherichia coli and Salmonella", 2. Ausgabe, S. 514-527). Bei CysP und Sbp handelt es sich um zwei verschiedene periplasmatische Bindeproteine. CysP besitzt eine höhere Affinität für Thiosulfat und Sbp fur Sulfat. CysA bildet die ATP-bindende Komponente. CysU und CysW sind die Transmembrankomponenten. In WO2009/043803, wurde bei *E. coli* die Expression des cys*PUWAM*-Operons verstärkt und dadurch eine Verbesserung der L-Methionin-Produktion erreicht. Bei *C. glutamicum* ist nichts über die Aufnahme von Thiosulfat bekannt. *Knock out* Mutanten des putativen Sulfat-Aufnahmesystems CysZ (cg3112) können noch mit Thiosulfat als S-Quelle wachsen (Rückert C. et al. (2005), BMC Genomics., Vol. 6(121)). CysZ ist somit nicht (alleine) für den Transport von Thiosulfat verantwortlich.

Die O-Acetylserin (thiol)-Lyase B (=CysM, EC 2.5.1.47) ermöglicht es *E. coli,* Thiosulfat als S-Quelle für die Synthese von L-Cystein und L-Methionin zu nutzen. Das Enzym katalysiert die Bildung von S-Sulfocystein (R-S-SO₃) und Acetat aus o-Acetylserin und Thiosulfat. S-Sulfocystein wird anschließend auf bisher nicht charakterisierte Weise zu L-Cystein und Sulfit oder Sulfat gespalten (Kredich N.M. (1996) in Neidhardt FC et al. (Hrsg.) "Escherichia coli and Salmonella", 2. Ausgabe, S. 514-527). Das Sulfat wird in drei Schritten durch die ATP-Sulfurylase (CysDN), die APS-Kinase (CysC) und die PAPS-Sulfotransferase (CysH) zu Sulfit (SO₃²⁻) reduziert. Anschließend wird Sulfit von der NADPH-Sulfitreductase weiter zu Sulfid (S²⁻) reduziert, welches von der o-Acetylserin-(Thiol)-Lyase A (CysK) und der o-Acetylserin-(Thiol)-Lyase B (CysM) zur Synthese eines zweiten L-Cystein-Moleküls verwendet werden kann. *C. glutamicum* kann in Minimalmedium mit Thiosulfat als S-Quelle wachsen (Rückert C. et al. (2005), BMC Genomics., Vol. 6(121)). Allerdings besitzt *C*. *glutamicum* keine O-Acetylserin (thiol)-Lyase B ("CysM") (Rückert und Kalinowski (2008) in A. Burkovski (ed.) "Corynebacteria: Genomics and Molecular Biology", Caister Academic Press). Thiosulfat muss daher auf andere Weise assimiliert werden.

Aufgabe der vorliegenden Erfindung ist es, ein Verfahren und Mikroorganismenstämme zur Verfügung zu stellen, die eine höhere Überproduktion von schwefelhaltigen Aminosäuren, insbesondere von L-Methionin ermöglicht.

Diese Aufgabe wird gelöst durch ein Verfahren zur fermentativen Herstellung schwefelhaltiger Aminosäuren ausgewählt aus der Gruppe L-Methionin, L-Cystein, L-Cystin, L-Homocystein und L-Homocystin, enthaltend die Schritte:
a) Bereitstellen eines Mikroorganismus der Familie Enterobacteriaceae oder eines Mikroorganismus der Familie Corynebacteriaceae, der ein Gen kodierend für ein Polypeptid mit der Aktivität einer Thiosulfat-Sulfurtransferase überexprimiert;
b) Fermentieren des Mikroorganismus aus a) in einem Medium, welches als anorganische Schwefelquelle ein Salz der Thioschwefelsäure oder Mischung aus einem Salz der Thioschwefelsäure und einem Salz der Schwefelsäure enthält, wobei eine Fermentationsbrühe erhalten wird, und
c) Anreichern der schwefelhaltigen Aminosäure in der Fermentationsbrühe aus b).

Bei Thiosulfat-Sulfurtransferasen, auch "Rhodanesen" genannt (EC 2.8.1.1), handelt es sich um Enzyme, welche das reduzierte Schwefelatom von Thiosulfat auf Cyanid (CN⁻) übertragen.

Einige Thiosulfat-Sulfurtransferasen können das reduzierte Schwefelatom auch auf alternative Substrate, z.B. Dihydrolipoat, übertragen (Alexander K., Volini M, 1987, J. Biol. Chem., 262: 6595-6604). In der Datenbank "Clusters of Orthologous Groups of proteins" (COG) finden sich in der Katogorie COG0607 "Rhodanese-related sulfurtransferase" zur Zeit 168 homologe Sulfurtransferasen in allen drei Domänen des Lebens (Tatusov RL, Fedorova ND, Jackson JD, Jacobs AR, Kiryutin B, Koonin EV, Krylov DM, Mazumder R, Mekhedov SL, Nikolskaya AN, Rao BS, Smirnov S, Sverdlov AV, Vasudevan S, Wolf YI, Yin JJ, Natale DA, 2003, BMC Bioinformatics, 4:41; http://www.ncbi.nlm.nih.gov/COG/). Rhodanesen besitzen eine oder mehrere charakteristische Rhodanese-ähnliche Domänen ("Rhodanese-like domain"; PFAM-Datenbank PF00581, http://pfam.sanger.ac.uk/; Gliubich F, Gazerro M, Zanotti G, Delbono S, Bombieri G, Berni R , 1996, J Biol Chem., 271(35):21054-61).

Das Gen RDL2 (Rhodanese-Like Protein) aus *Saccharomyces cerevisiae* S288c kodiert für das 149 Aminosäuren lange Protein Rdl2p. Es besitzt eine Rhodanese-ähnliche Domäne ("Rhodanese-like domain", PFAM-Datenbank PF00581, http://pfam.sanger.ac.uk/). Es wurde experimentell gezeigt, dass es sich bei Rdl2p um eine Thiosulfat-Sulfurtransferase (Rhodanese, EC 2.8.1.1) handelt (Foster MW, Forrester MT, Stamler JS, 2009, Proc Natl Acad Sci U S A, 106(45):18948-53). SEQ ID NO: 1 zeigt die DNA-Sequenz des Gens RDL2. SEQ ID NO: 2 zeigt die Aminosäuresequenz des durch RDL2 kodierten Proteins Rdl2p.

*E. coli* besitzt neben den gut charakterisierten Rhodanesen GlpE und PspE (Adams H, Teertstra W, Koster M, Tommassen J, 2002, FEBS Lett. 518:173-6) sieben weitere paraloge Enzyme mit Rhodanese-ähnlichen Domänen (Cheng H, Donahue JL, Battle SE, Ray WK, Larson TJ, 2008, Open Microbiol J., 2:18-28) :
YgaP hat eine N-terminale Rhodanese-ähnliche Domäne und zwei Transmembran-Domänen. Es zeigt *in vitro* Rhodanese-Aktivität (Ahmed, F., 2003, Dissertation).

YbbB ist eine tRNA 2-Selenouridin Synthase und hat eine N-terminale Rhodanese-ähnliche Domäne (Wolfe MD, Ahmed F, Lacourciere GM, Lauhon CT, Stadtman TC, Larson TJ, 2004, J Biol Chem., 279(3):1801-1809).

ThiI ist für die Synthese von Thiamin notwendig und spielt eine Rolle bei der Umsetzung von Uridin zu 4-Thiouridin an Position 8 in tRNA (Palenchar PM, Buck CJ, Cheng H, Larson TJ, Mueller EG, 2000, J Biol Chem., 275(12):8283-8286).

SseA ist eine 3-Mercaptopyruvat:Cyanid Sulfurtransferase, welche bevorzugt 3-Mercaptopyruvat statt Thiosulfat auf Cyanid überträgt (Colnaghi R, Cassinelli G, Drummond M, Forlani F, Pagani S, 2001, FEBS Lett., 500(3):153-156). Das Enzym hat zwei Rhodanese-ähnliche Domänen.

Der ORF ynjE kodiert für eine putative Sulfurtransferase mit drei Rhodanese-ähnlichen Domänen (Hänzelmann P, Dahl JU, Kuper J, Urban A, Müller-Theissen U, Leimkühler S, Schindelin H., 2009, Protein Sci., 18(12):2480-2491).

Der ORF yibN kodiert für eine putative Sulfurtransferase mit einer C-terminalen Rhodanese-ähnlichen Domäne.

Der ORF yceA kodiert für eine putative Sulfurtransferase mit einer Rhodanese-ähnlichen Domäne.

In *Corynebacterium glutamicum* kodieren mindestens 7 ORFs für mutmaßliche Sulfurtransferasen:
Der ORF thtR (=cg0803, NCgl0671) kodiert für eine putative Sulfurtransferase mit zwei Rhodanese-ähnlichen Domänen und einer Länge von 301 Aminosäuren.

Der ORF sseA2 (=cg1613, NCgl1369) kodiert für eine putative Sulfurtransferase mit zwei Rhodanese-ähnlichen Domänen und einer Länge von 289 Aminosäuren.

Der ORF cg3000 (=NCgl2616) kodiert für eine putative Sulfurtransferase mit einer Rhodanese-ähnlichen Domäne und einer Länge von 96 Aminosäuren.

Der ORF cg0073 (=NCgl0053) kodiert für eine putative Sulfurtransferase mit einer Rhodanese-ähnlichen Domäne und einer Länge von 97 Aminosäuren.

Der ORF cg0074 (=NCgl0054) kodiert für eine putative Sulfurtransferase mit einer Rhodanese-ähnlichen Domäne und einer Länge von 197 Aminosäuren.

Der ORF sseA1 (=cg3073, NCgl2678) kodiert für eine putative Sulfurtransferase mit zwei Rhodanese-ähnlichen Domänen und einer Länge von 274 Aminosäuren.

Der ORF cg3319 (=NCgl2890) kodiert für eine putative Sulfurtransferase mit einer Rhodanese-ähnlichen Domäne und einer Länge von 312 Aminosäuren.

Die Thiosulfat-Sulfurtransferase aus dem Rind (*Bos taurus*) besitzt zwei Rhodanese-ähnliche Domänen und ist gut charakterisiert (Cannella C, Costa M, Pensa B, Ricci G, Pecci L, Cavallini D., 1981, Eur J Biochem., 119(3):491-495).

In einer bevorzugten Ausführungsform des Verfahrens überexprimiert der Mikroorganismus ein oder mehrere Gen(e) kodierend für ein Polypeptid mit der Aktivität einer Thiosulfat-Sulfurtransferase, wobei das Polypeptid mit der Aktivität einer Thiosulfat-Sulfurtransferase ausgewählt ist aus den folgenden a) bis d):
a) ein Polypeptid bestehend aus oder enthaltend die Polypeptide Rdl2p, GlpE, PspE, YgaP, ThiI, YbbB, SseA, YnjE, YceA, YibN, NCgl0671, NCgl1369, NCgl2616, NCgl0053, NCgl0054, NCgl2678, NCgl2890; Thiosulfat-Sulfurtransferase aus Säugetieren, beispielsweise die Thiosulfat-Sulfurtransferase aus der Rinderleber (*Bos taurus*); bevorzugt Rdl2p, GlpE, PspE und besonders bevorzugt Rdl2p;
b) ein Polypeptid bestehend aus oder enthaltend die in SEQ ID NO: 2 gezeigte Aminosäuresequenz;
c) ein Polypeptid mit einer Aminosäuresequenz, die zu 70% oder mehr identisch ist mit der Aminosäuresequenz von a) oder b), wobei das Polypeptid Thiosulfat-Sulfurtransferase-Aktivität aufweist;
d) ein Polypeptid, das eine Aminosäuresequenz enthaltend eine Deletion, Substitution, Insertion und/oder Addition von 1 bis 45 Aminosäureresten bezüglich der in SEQ ID NO: 2 gezeigten Aminosäuresequenz aufweist, wobei das Polypeptid Thiosulfat-Sulfurtransferase-Aktivität aufweist.

Wie oben erwähnt, umfassen Polypeptide mit der Aktivität einer Thiosulfat-Sulfurtransferase auch Varianten der unter a) bzw. b) genannten Enzyme, mit einer Aminosäuresequenz, die zu 70% oder mehr identisch ist mit der Aminosäuresequenz der unter a) bzw. b) genannten Sequenzen, wobei die Varianten Thiosulfat-Sulfurtransferase-Aktivität aufweisen. In bevorzugten Ausführungsformen sind Varianten umfasst, die wenigstens 75%, wenigstens 80%, wenigstens 85%, wenigstens 90%, wenigstens 95%, wenigstens 98% oder wenigstens 99% identisch zu den oben beschriebenen Aminosäuresequenzen sind, d.h. wobei wenigstens 75%, wenigstens 80%, wenigstens 85%, wenigstens 90%, wenigstens 95%, wenigstens 98% oder wenigstens 99% der Aminosäurepositionen identisch zu jenen der oben beschriebenen Aminosäuresequenzen sind. Die prozentuale Identität wird vorzugsweise über die gesamte Länge der Aminosäure oder Nukleinsäureregion berechnet. Eine Reihe von Programmen, die auf einer Vielzahl von Algorithmen beruhen, steht dem Fachmann für den Sequenzvergleich zur Verfügung. In diesem Zusammenhang ergeben die Algorithmen von Needleman und Wunsch oder Smith und Waterman besonders verlässliche Ergebnisse. Für das Alignment der Sequenzen stehen das Programm PileUp (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970)) und Smith and Waterman (Adv. Appl. Math. 2; 482-489 (1981))], die zum Softwarepaket GCG gehören [Genetics Computer Group, 575 Science Drive, Madison, Wisconsin, USA 53711 (1991)] zur Verfügung. Die oben aufgeführten Prozentwerte für die Sequenzidentität werden vorzugsweise mit dem Programm GAP über die gesamte Sequenzregion berechnet.

Weiterhin umfassen Polypeptide mit der Aktivität einer Thiosulfat-Sulfurtransferase auch Fragmente der in a) bzw. b) genannten Enzyme. Diese Fragmente weisen die oben beschriebene Aktivität auf. Ein Fragment eines Polypeptides mit der Aktivität einer Thiosulfat-Sulfurtransferase im Sinne dieser Patentanmeldung enthält vorzugsweise wenigstens 30, wenigstens 50, oder wenigstens 80 aufeinander folgende Aminosäurereste einer der vorgenannten Aminosäuresequenzen.

Wie oben erwähnt, umfassen Polypeptide mit der Aktivität einer Thiosulfat-Sulfurtransferase auch Varianten der unter a) bzw. b) genannten Enzyme, die eine Aminosäuresequenz enthaltend eine Deletion, Substitution, Insertion und/oder Addition von 1 bis 45 Aminosäureresten bezüglich der in SEQ ID NO: 2 gezeigten Aminosäuresequenz aufweisen, wobei das Polypeptid Thiosulfat-Sulfurtransferase-Aktivität aufweist. In bevorzugten Ausführungsformen enthält die Aminosäuresequenz eine Deletion, Substitution, Insertion und/oder Addition von 1 bis 40, weiter bevorzugt von 1 bis 30, noch weiter bevorzugt von 1 bis 20, vorzugsweise von 1 bis 15, noch weiter bevorzugt von 1 bis 10 und am meisten bevorzugt von 1 bis 5 Aminosäureresten bezüglich der in SEQ ID NO: 2 gezeigten Aminosäuresequenz.

In einem weiteren bevorzugten Verfahren wird das Polypeptid von einem Gen kodiert, das die Nukleotidsequenz von SEQ ID NO:1 umfasst, die der Wildtyp-Sequenz des Gens RDL2 aus *Saccharomyces cerevisiae* S288c entspricht.

Weiterhin ist bevorzugt, das Polypeptid von einem Gen kodiert wird, das die Nukleotidsequenz von SEQ ID NO:3 umfasst. Die Kodon-Verwendung ist hier leicht an jene von *E.coli* angepasst.

In einem weiteren bevorzugten Verfahren wird das Polypeptid von einem Gen, das die Nukleotidsequenz von SEQ ID NO:4 umfasst. Die Kodon-Verwendung ist hier stärker an jene von *E.coli* angepasst.

Weiterhin ist bevorzugt, dass das Polypeptid von einem Gen kodiert wird, das die Nukleotidsequenz von SEQ ID NO:5 umfasst. Die Kodon-Verwendung ist hier vollständig an jene von *E.coli* angepasst.

Bei der Durchführung des erfindungsgemäßen Verfahrens, wird vorzugsweise die Expression des Gens kodierend für ein Polypeptid mit der Aktivität einer Thiosulfat-Sulfurtransferase durch eine oder mehrere der folgenden Maßnahmen erhöht:
a) Die Expression des Gens steht unter der Kontrolle eines Promotors, der in dem für das Verfahren verwendeten Mikroorganismus zu einer gegenüber dem Ausgangsstamm verstärkten Expression führt. Dabei kann beispielsweise ein konstitutiver GAPDH-Promoter des gapA-Gens von *Escherichia coli,* oder ein induzierbarer lac-, tac-, trc-, lambda-, ara oder tet-Promotor verwendet werden (Review: Makrides SC. Micro-biol Rev. 1996 Sep; 60(3):512-38).
b) Die Kopienzahl des Gens kodierend für ein Polypeptid mit der Aktivität einer Thiosulfat-Sulfurtransferase wird gegenüber dem Ausgangsstamm erhöht. Dies kann beispielsweise erreicht werden, indem das Gen in Plasmide mit erhöhter Kopienzahl eingefügt wird, oder indem das Gen in das Chromosom des Mikroorganismus in mehreren Kopien integriert wird (Baneyx F, 1999, Curr. Opin. Biotechnol. 10, 411-421).
c) Die Expression des Gens erfolgt unter Verwendung einer Ribosomen-Bindestelle, die in dem für das Verfahren verwendeten Mikroorganismus zu einer gegenüber dem Ausgangsstamm verstärkten Translation führt (Makrides SC. Microbiol Rev. 1996 Sep;60(3):512-38).
d) Die Expression des Gens wird durch eine Optimierung der Kodon-Verwendung (codon usage) des Gens bezogen auf den für das Verfahren verwendeten Mikroorganismus verstärkt (Welch, Villalobos, Gustafsson and Minshull, J R Soc Interface, 2009, 6:5467-76). Als Maß für die Anpassung der Kodon-Verwendung eines Gens an einen Organismus ist beispielswei-se der "Codon Adaptation Index" (CAI) geeignet (Sharp PM, Li WH, 1987, Nucleic Acids Res., 15(3):1281-95).
e) Die Expression des Gens wird durch die Reduzierung von mRNA Sekundärstrukturen in der vom Gen transkribierten mRNA verstärkt (Kudla G, Murray AW, Tollervey D, Plotkin JB., Science, 2009 Apr 10;324(5924):255-8; Welch, Villalobos, Gustafsson and Minshull, J R Soc Interface, 2009, 6:5467-76).
f) Die Expression des Gens wird durch die Eliminierung von RNA-Polymerase-Terminatoren in der vom Gen transkribierten mRNA verstärkt (Welch, Villalobos, Gustafsson and Minshull, J R Soc Interface, 2009, 6:S467-76).
g) Die Expression des Gens erfolgt unter Verwendung mRNAstabilisierender Sequenzen in der vom Gen transkribierten mRNA (Carrier TA, Keasling JD, Biotechnol Prog., 1997, Nov-Dec; 13(6):699-708). Als Beispiel kann die Sequenz ARNmst17 zur Stabilisierung der mRNA des Gens metF von *E. coli* genannt werden (WO2009/043803).

In einer weiteren Ausführungsform des erfindungsgemäßen Verfahrens ist bevorzugt, dass es sich bei dem Salz der Thioschwefelsäure um ein Salz ausgewählt aus der Gruppe Alkalisalz, Erdalkalisalz, Ammoniumsalz und Mischungen davon, bevorzugt Ammoniumsalz, handelt.

Weiterhin ist bevorzugt, dass es sich bei dem Salz der Schwefelsäure um ein Salz ausgewählt aus der Gruppe Alkalisalz, Erdalkalisalz, Ammoniumsalz (und Mischungen), bevorzugt Ammoniumsalz, handelt.

Vorzugsweise beträgt die Konzentration des Salzes der Thioschwefelsäure in dem Medium bzw. in der Fermentationsbrühe 0,05 g/kg bis 100 g/kg, bevorzugt 0,1 g/kg bis 20 g/kg und besonders bevorzugt 0,2 g/kg bis 12 g/kg.

In einem bevorzugten Verfahren wird die Konzentration des Salzes der Thioschwefelsäure in dem Medium bzw. in der Fermentationsbrühe während der Fermentation auf mindestens 0,05 g/kg bis 100 g/kg, bevorzugt 0,1 g/kg bis 20 g/kg und besonders bevorzugt 0,2 g/kg bis 12 g/kg gehalten.

In einem weiteren bevorzugten Verfahren wird während der Fermentation der Anteil des Salzes der Thioschwefelsäure bezogen auf den Gesamtgehalt an anorganischem Schwefel im Medium und in der Fermentationsbrühe auf mindestens 5 mol% gehalten.

Das erfindungsgemäße Verfahren kann als batch-Verfahren, fed batch-Verfahren, repeated fed batch Verfahren und kontinuierliches Verfahren ausgestaltet sein.

Weiterhin kann die schwefelhaltige Aminosäure aus der Fermentationsbrühe als festes Produkt oder gelöst in einem flüssigen Produkt gewonnen werden.

In einem bevorzugten Verfahren der vorliegenden Erfindung handelt es sich bei der zu produzierenden schwefelhaltigen Aminosäure um L-Methionin.

Weiterhin ist bevorzugt, dass es sich bei dem Mikroorganismus um die Gattung *Escherichia,* besonders bevorzugt um die Art *Escherichia coli,* handelt.

In einem alternativen Verfahren handelt es sich bei dem Mikroorganismus um die Gattung *Corynebacterium,* besonders bevorzugt um die Art *Corynebacterium glutamicum.*

In einem besonders bevorzugten Verfahren der vorliegenden Erfindung ist der Mikroorganismus ausgewählt aus:
- *Corynebacterium glutamicum* mit erhöhter Aktivität und/oder Expression der Aspartatkinase und Abschwächung oder Deletion des Regulatorproteins McbR im Vergleich zum Ausgangsstamm;
- *Escherichia coli,* mit erhöhter Aktivität und/oder Expression der Aspartatkinase und Abschwächung oder Deletion des Regulatorproteins MetJ im Vergleich zum Ausgangsstamm.

Darüber hinaus stellt die Erfindung einen Mikroorganismus bereit ausgewählt aus
- *Corynebacterium glutamicum* mit erhöhter Aktivität und/oder Expression der Aspartatkinase und Abschwächung oder Deletion des Regulatorproteins McbR im Vergleich zum Ausgangsstamm;
- *Escherichia coli,* mit erhöhter Aktivität und/oder Expression der Aspartatkinase und Abschwächung oder Deletion des Regulatorproteins MetJ im Vergleich zum Ausgangsstamm; wobei der Mikroorganismus L-Methionin ausscheidet oder produziert, und wobei der Mikroorganismus ein Gen kodierend für ein Polypeptid mit der Aktivität einer Thiosulfat-Sulfurtransferase überexprimiert.

Der L-Methionin ausscheidende oder produzierende Stamm des *C. glutamicum* weist bevorzugt eine verstärkte Enzymaktivität der Aspartatkinase (EC 2.7.2.4) auf, wobei feedbackresistente Allele bevorzugt sind. Bei Corynebacterium wird diese Aspartatkinase von dem Gen *lysC* kodiert. Weiterhin erfolgt durch die Abschwächung oder Deletion des Regulatorproteins McbR (das von dem Gen *mcbR* kodiert wird) eine Steigerung der Schwefelverwertung. McbR stellt den Repressor der gesamten Schwefelverwertungskaskade in *C*. *glutamicum* dar (Rey DA, Nentwich SS, Koch DJ, Rückert C, Pühler A, Tauch A, Kalinowski J., Mol Microbiol., 2005,56(4):871-887).

Der L-Methionin ausscheidende oder produzierende Stamm des *E. coli* weist bevorzugt eine verstärkte Enzymaktivität der Aspartatkinase (EC 2.7.2.4) auf, wobei feedbackresistente Allele bevorzugt sind. In *E. coli* gibt es drei verschiedene Aspartatkinasen, die von den Genen *thrA, metL* oder *lysC* kodiert werden. Weiterhin erfolgt durch die Abschwächung oder Deletion des Regulatorproteins MetJ, das von dem Gen *metJ* kodiert wird eine Steigerung der L-Methionin-Biosynthese. MetJ stellt den Hauptrepressor der L-Methionin-Biosynthese in *E. coli* dar.

Weiterhin ist bevorzugt, dass dieser Mikroorganismus ein oder mehrere Gen(e) kodierend für ein Polypeptid mit der Aktivität einer Thiosulfat-Sulfurtransferase überexprimiert, wobei das Polypeptid mit der Aktivität einer Thiosulfat-Sulfurtransferase ausgewählt ist aus den folgenden a) bis d) :
a) ein Polypeptid bestehend aus oder enthaltend die Polypeptide Rdl2p, GlpE, PspE, YgaP, ThiI, YbbB, SseA, YnjE, YceA, YibN, NCgl0671, NCgl1369, NCgl2616, NCgl0053, NCgl0054, NCgl2678, NCgl2890; Thiosulfat-Sulfurtransferase aus Säugetieren, beispielsweise die Thiosulfat-Sulfurtransferase aus der Rinderleber (*Bos taurus*); bevorzugt Rdl2p, GlpE, PspE und besonders bevorzugt Rdl2p;;
b) ein Polypeptid bestehend aus oder enthaltend die in SEQ ID NO: 2 gezeigte Aminosäuresequenz;
c) ein Polypeptid mit einer Aminosäuresequenz, die zu 70% oder mehr identisch ist mit der Aminosäuresequenz von a) oder b), wobei das Polypeptid Thiosulfat-Sulfurtransferase-Aktivität aufweist;
d) ein Polypeptid, das eine Aminosäuresequenz enthaltend eine Deletion, Substitution, Insertion und/oder Addition von 1 bis 45 Aminosäureresten bezüglich der in SEQ ID NO: 2 gezeigten Aminosäuresequenz aufweist, wobei das Polypeptid Thiosulfat-Sulfurtransferase-Aktivität aufweist.

Weiterhin ist der Ausgangsstamm des Mikroorganismus bevorzugt abgeleitet aus der Gruppe bestehend aus *Escherichia coli* MG1655, *Escherichia coli* W3110, *Escherichia coli* DH5α, *Escherichia coli* DH10B, *Escherichia coli* BW2952, *Escherichia coli* REL606, *Corynebacterium glutamicum* ATCC13032, *Corynebacterium glutamicum* R, *Corynebacterium glutamicum* DSM20411 (alte Bezeichnung *Brevibacterium flavum*), *Corynebacterium glutamicum* DSM20412 (alte Bezeichnung *Brevibacterium lactofermentum*), *Corynebacterium glutamicum* DSM1412 (alte Bezeichnung *Brevibacterium lactofermentum*), *Corynebacterium efficiens* YS-314^{T} (=DSM44549), *Corynebacterium glutamicum* ATCC21608, *Corynebacterium glutamicum* DSM17322.

Ein weiterer L-Methionin ausscheidender bzw. produzierender Stamm ist beispielsweise der *E. coli* Produktionsstamm MG1655 ΔmetJ Ptrc-metH Ptrc-metF PtrcF-cysPUWAM PtrcF-cysJIH Ptrc09-gcvTHP enthaltend das Produktionsplasmid pME101-thrA*1-cysE-Pgap-metA*11 (WO2009/043803).

Die Klonierung des *E. coli* Produktionsstammes MG1655ΔmetJ Ptrc-metH Ptrc-metF PtrcF-cysPUWAM PtrcF-cysJIH Ptrc09-gcvTHP wird in der Patentanmeldung WO2009/043803 beschrieben. Der Stamm basiert auf dem Wildtypstamm *E. coli* K12 MG1655. Im Genom dieses Stammes wurden die folgenden Veränderungen eingeführt:
- Das Gen für den Repressor dem L-Methioninbiosynthese *metJ* wurde deletiert.
- Upstream des Gens *metH* (kodiert für die Cobalaminabhängige Methioninsynthase) wurde der starke trc-Promoter inseriert.
- Upstream des Gens *metF* (kodiert für die 5,10-Methylentetrahydrofolat-Reduktase) wurde der starke trc-Promoter inseriert.
- Upstream des Operons *cysPUWAM* wurde der starke trcF-Promoter inseriert. *cysPUWA* kodiert für einen Sulfat/Thiosulfat-Aufnahmetransporter. *cysM* kodiert für die Cystein-Synthase B.
- Upstream des Operons *cysJIH* wurde der starke trcF-Promoter inseriert. *cysJI* kodiert für die SulfitReductase und *cysH* kodiert für die 3'-Phospho-Adenylylsulfat-Reduktase.
- Upstream des Operons *gcvTHP* wurde der starke trc09-Promoter inseriert. *gcvT, gcvH* und *gcv P* kodieren für drei Komponenten des Glycin-Cleavage-Systems.

Die Klonierung des *E. coli* Produktionsplasmides pME101-thrA*1-cysE-Pgap-metA*11 wird in der Patentanmeldung WO2007/077041 beschrieben. Es handelt sich um ein Plasmid niedriger Kopienzahl (*low copy* plasmid) auf Basis des Vektors pCL1920 (Lerner, C.G. and Inouye, M., Nucl. Acids Res. (1990) 18:4631 [PMID: 2201955]). Das Leerplasmid pME101 besitzt das *lacI^{q}*-Gen, welches für ein stark exprimiertes Allel des lac-Repressors kodiert. Downstream eines starken, durch den Lac-Repressor reprimierbaren trc-Promoters wurde das Gen *thrA***1* kloniert. Es kodiert für eine *feedback-*resistente Variante der Aspartatkinase / Homoserindehydrogenase ThrA aus *E. coli.* In der gleichen Orientierung dahinter liegt das Gen *cysE* mitsamt seinem natürlichen Promoter. Es kodiert für die Serin-Acetyltransferase aus *E. coli.* Downstream von *cysE* folgt der starke *gapA*-Promoter aus *E. coli,* welcher die Expression des Gens *metA*11* kontrolliert. *metA*11* kodiert für eine *feedback*-resistente Variante der Homoserin O-Succinyltransferase aus *E. coli.*

Als Beispiele für weitere L-Methionin ausscheidende bzw. produzierende Mikroorganismen können folgende Stämme genannt werden:
- *C*. *glutamicum* M1179 (=DSM17322) (WO2007/011939)
- *E. coli* TF4076BJF metA#10 + metYX(Lm) (WO2008/127240; Seite 46);
- *E. coli* W3110ΔJ/pKP451 (EP 1 445 310 B1, Seite 7 Bsp. 4)
- *E. coli* WΔthrBCΔmetJmetK32 pMWPthrmetA4Δ5Δ9 (Yoshihiro Usuda and Osamu Kurahashi, 2005, Applied and Environmental Microbiology, Vol. 71, No. 6, p. 3228-3234)
- W3110/pHC34 (WO01/27307 Seite 13, Bsp. 3).

Weitere Beispiele verschiedener geeigneter Mikroorganismen sind bei Gomes et al. beschrieben (Enzyme and Microbial Technology 37 (2005), 3-18).

L-Methionin produzierende Bakterien können eines oder mehrere der Merkmale ausgewählt aus der folgenden Gruppe besitzen:
1) überexprimiertes Polynukleotid, das für eine oder mehrere Komponenten des Thiosulfat-/Sulfat-Transportsystems CysPUWA (EC 3.6.3.25) kodiert,
2) überexprimiertes Polynukleotid, das für eine 3'-Phosphoadenosin 5'-Phosphosulfat Reduktase CysH (EC 1.8.4.8) kodiert,
3) überexprimiertes Polynukleotid, das für eine oder mehrere Komponenten der Sulfit Reduktase CysJI (EC 1.8.1.2) kodiert,
4) überexprimiertes Polynukleotid, das für eine Cystein Synthase A CysK (EC 2.5.1.47) kodiert,
5) überexprimiertes Polynukleotid, das für eine Cystein Synthase B CysM (EC 2.5.1.47) kodiert,
6) überexprimiertes Polynukleotid, das für eine Serin Acetyltransferase CysE (EC 2.3.1.30) kodiert,
7) überexprimiertes Polynukleotid, das für eine oder mehrere Komponenten des Glycin Cleavage Systems GcvTHP-Lpd (EC 2.1.2.10, EC 1.4.4.2, EC 1.8.1.4) kodiert,
8) überexprimiertes Polynukleotid, das für eine Lipoyl Synthase LipA (EC 2.8.1.8) kodiert,
9) überexprimiertes Polynukleotid, das für eine Lipoyl-Protein Ligase LipB (EC 2.3.1.181) kodiert,
10) überexprimiertes Polynukleotid, das für eine Phosphoglycerat Dehydrogenase SerA (EC 1.1.1.95) kodiert,
11) überexprimiertes Polynukleotid, das für eine 3-Phosphoserin Phosphatase SerB (EC 3.1.3.3) kodiert,
12) überexprimiertes Polynukleotid, das für eine 3-Phosphoserin/Phosphohydroxythreonin Aminotransferase SerC (EC 2.6.1.52) kodiert,
13) überexprimiertes Polynukleotid, das für eine Serin Hydroxymethyltransferase GlyA (EC 2.1.2.1) kodiert,
14) überexprimiertes Polynukleotid, das für eine Aspartokinase I und Homoserin Dehydrogenase I ThrA (EC 2.7.2.4, EC 1.1.1.3) kodiert,
15) überexprimiertes Polynukleotid, das für eine Aspartatkinase LysC (EC 2.7.2.4) kodiert,
16) überexprimiertes Polynukleotid, das für eine Homoserin-Dehydrogenase Hom (EC 1.1.1.3) kodiert,
17) überexprimiertes Polynukleotid, das für eine Homoserin O-Acetyltransferase MetX (EC 2.3.1.31) kodiert,
18) überexprimiertes Polynukleotid, das für eine Homoserin O-Succinlytransferase MetA (EC 2.3.1.46) kodiert,
19) überexprimiertes Polynukleotid, das für eine Cystathionin gamma-Synthase MetB (EC 2.5.1.48) kodiert,
20) überexprimiertes Polynukleotid, das für eine β-C-S-Lyase AecD (EC 4.4.1.8, auch als beta-Lyase bezeichnet) kodiert,
21) überexprimiertes Polynukleotid, das für eine Cystathionin beta-Lyase MetC (EC 4.4.1.8) kodiert,
22) überexprimiertes Polynukleotid, das für eine B12 unabhängige Homocystein S-Methyltransferase MetE (EC 2.1.1.14) kodiert,
23) überexprimiertes Polynukleotid, das für eine B12 abhängige Homocystein S-Methyltransferase MetH (EC 2.1.1.13) kodiert,
24) überexprimiertes Polynukleotid, das für eine Methylentetrahydrofolat Reduktase MetF (EC 1.5.1.20) kodiert,
25) überexprimiertes Polynukleotid, das für eine oder mehrere Komponenten des L-Methionin Exporters BrnFE aus *Corynebacterium glutamicum* kodiert,
26) überexprimiertes Polynukleotid, das für eine oder mehrere Komponenten des Valin Exporter YgaZH aus *Escherichia coli* (b2682, b2683) kodiert,
27) überexprimiertes Polynukleotid, das für den putativen Transporter YjeH aus *Escherichia coli* (b4141) kodiert,
28) überexprimiertes Polynukleotid, das für eine oder mehrere Komponenten der Pyridin Nucleotid Transhydrogenase PntAB (EC 1.6.1.2) kodiert,
29) überexprimiertes Polynukleotid, das für eine O-Succinylhomoserine Sulfhydrylase MetZ (EC 2.5.1.48) kodiert,
30) überexprimiertes Polynukleotid, das für eine Phosphoenolpyruvate Carboxylase Pyc (EC 4.1.1.31) kodiert.

Bevorzugte Merkmale sind hierbei eine oder mehrere ausgewählt aus der Gruppe:
1) überexprimiertes Polynukleotid, das für eine oder mehrere Komponenten des Thiosulfat-/Sulfat-Transportsystems CysPUWA (EC 3.6.3.25) kodiert,
2) überexprimiertes Polynukleotid, das für eine 3'-Phosphoadenosin 5'-Phosphosulfat Reduktase CysH (EC 1.8.4.8) kodiert,
3) überexprimiertes Polynukleotid, das für eine oder mehrere Komponenten der Sulfit Reduktase CysJI (EC 1.8.1.2) kodiert,
4) überexprimiertes Polynukleotid, das für eine Cystein Synthase A CysK (EC 2.5.1.47) kodiert,
5) überexprimiertes Polynukleotid, das für eine Cystein Synthase B CysM (EC 2.5.1.47) kodiert,
6) überexprimiertes Polynukleotid, das für eine Serin Acetyltransferase CysE (EC 2.3.1.30) kodiert,
7) überexprimiertes Polynukleotid, das für eine oder mehrere Komponenten des Glycin Cleavage Systems GcvTHP-Lpd (EC 2.1.2.10, EC 1.4.4.2, EC 1.8.1.4) kodiert,
8) überexprimiertes Polynukleotid, das für eine Lipoyl Synthase LipA (EC 2.8.1.8) kodiert,
9) überexprimiertes Polynukleotid, das für eine Lipoyl-Protein Ligase LipB (EC 2.3.1.181) kodiert,
10) überexprimiertes Polynukleotid, das für eine Phosphoglycerat Dehydrogenase SerA (EC 1.1.1.95) kodiert,
11) überexprimiertes Polynukleotid, das für eine 3-Phosphoserin Phosphatase SerB (EC 3.1.3.3) kodiert,
12) überexprimiertes Polynukleotid, das für eine 3-Phosphoserin/Phosphohydroxythreonin Aminotransferase SerC (EC 2.6.1.52) kodiert,
13) überexprimiertes Polynukleotid, das für eine Serin Hydroxymethyltransferase GlyA (EC 2.1.2.1) kodiert,
14) überexprimiertes Polynukleotid, das für eine Aspartokinase I und Homoserin Dehydrogenase I ThrA (EC 2.7.2.4, EC 1.1.1.3) kodiert,
15) überexprimiertes Polynukleotid, das für eine Aspartatkinase LysC (EC 2.7.2.4) kodiert,
16) überexprimiertes Polynukleotid, das für eine Homoserin-Dehydrogenase Hom (EC 1.1.1.3) kodiert,
17) überexprimiertes Polynukleotid, das für eine Homoserin-Acetyltransferase MetX (EC 2.3.1.31) kodiert,
18) überexprimiertes Polynukleotid, das für eine Homoserin O-Transsuccinylase MetA (EC 2.3.1.46) kodiert,
19) überexprimiertes Polynukleotid, das für eine Cystathionin gamma-Synthase MetB (EC 2.5.1.48) kodiert,
20) überexprimiertes Polynukleotid, das für eine β-C-S-Lyase AecD (EC 4.4.1.8, auch als beta-Lyase bezeichnet) kodiert,
21) überexprimiertes Polynukleotid, das für eine Cystathionin beta-Lyase MetC (EC 4.4.1.8) kodiert,
22) überexprimiertes Polynukleotid, das für eine B12 unabhängige Homocystein S-Methyltransferase MetE (EC 2.1.1.14) kodiert,
23) überexprimiertes Polynukleotid, das für eine B12 abhängige Homocystein S-Methyltransferase MetH (EC 2.1.1.13) kodiert,
24) überexprimiertes Polynukleotid, das für eine Methylentetrahydrofolat Reduktase MetF (EC 1.5.1.20) kodiert.

Ganz besonders bevorzugte Merkmale sind hierbei ausgewählt aus der Gruppe:
1) überexprimiertes Polynukleotid, das für eine Aspartokinase I und Homoserin Dehydrogenase I ThrA (EC 2.7.2.4, EC 1.1.1.3) kodiert,
2) überexprimiertes Polynukleotid, das für eine Aspartatkinase LysC (EC 2.7.2.4) kodiert,
3) überexprimiertes Polynukleotid, das für eine Homoserin-Dehydrogenase Hom (EC 1.1.1.3) kodiert,
4) überexprimiertes Polynukleotid, das für eine Homoserin-Acetyltransferase MetX (EC 2.3.1.31) kodiert,
5) überexprimiertes Polynukleotid, das für eine Homoserin O-Transsuccinylase MetA (EC 2.3.1.46) kodiert,
6) überexprimiertes Polynukleotid, das für eine Cystathionin gamma-Synthase MetB (EC 2.5.1.48) kodiert,
7) überexprimiertes Polynukleotid, das für eine β-C-S-Lyase AecD (EC 4.4.1.8, auch als beta-Lyase bezeichnet) kodiert,
8) überexprimiertes Polynukleotid, das für eine Cystathionin beta-Lyase MetC (EC 4.4.1.8) kodiert,
9) überexprimiertes Polynukleotid, das für eine B12 unabhängige Homocystein S-Methyltransferase MetE (EC 2.1.1.14) kodiert,
10) überexprimiertes Polynukleotid, das für eine B12 abhängige Homocystein S-Methyltransferase MetH (EC 2.1.1.13) kodiert,
11) überexprimiertes Polynukleotid, das für eine Methylentetrahydrofolat Reduktase MetF (EC 1.5.1.20) kodiert.

Für die Verbesserung der Produktion von L-Methionin in *C.glutamicum* ist es gegebenenfalls zweckmäßig, eines oder mehrere der Gene abzuschwächen, ausgewählt aus der Gruppe:
a) ein für die Glucose-6-Phosphat-Isomerase (Pgi, EC Nr. 5.3.1.9) kodierendes Gen pgi,
b) ein für die Homoserinkinase (ThrB, EC Nr. 2.7.1.39) kodierendes Gen thrB,
c) ein für die S-Adenosylmethionin Synthase (MetK, EC Nr. 2.5.1.6) kodierendes Gen metK,
d) ein für die Dihydrodipicolinat Synthase (DapA, EC Nr. 4.2.1.52) kodierendes Gen dapA,
e) ein für die Phosphoenolpyruvat Carboxykinase (Pck, EC Nr. 4.1.1.49) kodierendes Gen pck,
f) ein für die Cystathionin-γ-Lyase (Cg3086, EC Nr. 4.4.1.1)kodierendes Gen cg3086,
g) ein für die Cystathionin-β-Synthase (Cg2344, EC Nr. 4.2.1.22)kodierendes Gen cg2344,
h) ein für das Regulatorprotein Cg3031 kodierendes Gen cg3031,
i) ein für den Transkriptionsregulator der L-Methioninbiosynthese (McbR) kodierendes Gen mcbR,
j) ein für eine Untereinheit des L-Methionintransporters (MetQNI) kodierendes Gen metQ,
k) ein für eine Untereinheit des L-Methionintransporters (MetQNI) kodierendes Gen metN,
l) ein für eine Untereinheit des L-Methionintransporters (MetQNI) kodierendes Gen metI,
m) ein für den L-Methionintransporter (MetP) kodierendes Gen metP.

Für die Verbesserung der Produktion von L-Methionin in *E.coli* ist es gegebenenfalls zweckmäßig, eines oder mehrere der Gene abzuschwächen, ausgewählt aus der Gruppe:
a) ein für den Transkriptionsregulator der L-Methioninbiosynthese (MetJ) kodierendes Gen metJ (b3938, ECK3930),
b) ein für die Glucose-6-Phosphat-Isomerase (Pgi, EC Nr. 5.3.1.9) kodierendes Gen pgi (b4025, ECK4017),
c) ein für die Homoserinkinase (ThrB, EC 2.7.1.39) kodierendes Gen thrB (b0003, ECK0003),
d) ein für die S-Adenosylmethionin Synthase (MetK, EC Nr. 2.5.1.6) kodierendes Gen metK (b2942, ECK2937),
e) ein für die Dihydrodipicolinat Synthase (DapA, EC Nr. 4.2.1.52) kodierendes Gen dapA (b2478, ECK2474),
f) ein für die Phosphoenolpyruvat Carboxykinase (Pck, EC Nr. 4.1.1.49) kodierendes Gen pck (b3403, ECK3390),
g) ein für die Formyltetrahydrofolat Hydrolase (PurU, EC Nr. 3.5.1.10) kodierendes Gen purU (b1232, ECK1227),
h) ein für die Pyruvatkinase II (PykA, EC Nr. 2.7.1.40) kodierendes Gen pykA (b1854, ECK1855)
i) ein für die Pyruvatkinase I (PykF, EC 2.7.1.40) kodierendes Gen pykF (b1676, ECK1672),
j) ein für eine Untereinheit des L-Methionintransporters (MetQNI) kodierendes Gen metQ (b0197, ECK0197),
k) ein für eine Untereinheit des L-Methionintransporters (MetQNI) kodierendes Gen metI (b0198, ECK0198),
l) ein für eine Untereinheit des L-Methionintransporters (MetQNI) kodierendes Gen metN (b0199, ECK0199),
m) ein für die Deoxycytidin 5'-Triphosphat Deaminase (Dcd, EC Nr. 3.5.4.13) kodierendes Gen dcd (b2065, ECK2059),
n) ein für die putative N-Acyltransferase (YncA, Metabolic Explorer WO2010/020681) kodierendes Gen yncA (b1448, ECK1442),
o) ein für die regulatorische sRNA FnrS kodierendes Gen fnrS (b4699, ECK4511).

### Detaillierte Beschreibung der Erfindung

Die Erfindung betrifft ein Verfahren zur fermentativen Herstellung schwefelhaltiger Aminosäuren ausgewählt aus der Gruppe L-Methionin, L-Cystein, L-Cystin, L-Homocystein und L-Homocystin. Das Verfahren wird mit einem Mikroorganismus der Familie Enterobacteriaceae oder mit einem Mikroorganismus der Familie Corynebacteriaceae durchgeführt, der ein Gen kodierend für ein Polypeptid mit der enzymatischen Aktivität einer Thiosulfat-Sulfurtransferase überexprimiert.

Der Begriff "Gen" bedeutet hierbei ein Abschnitt auf der Desoxyribonukleinsäure (DNA), der die Informationen zur Herstellung (Transkription) zunächst einer Ribonukleinsäure (RNA) und diese die Information zur Herstellung (Translation) eines Proteins (Polypeptids) enthält, hier ein Polypeptid mit der Aktivität einer Thiosulfat-Sulfurtransferase. Die Tatsache, dass ein Gen oder ein Polynukleotid die Informationen zur Herstellung eines Proteins enthält, bezeichnet man auch als Kodierung eines Protein bzw. Polypeptides durch das Gen bzw. durch die RNA. Unter endogenen Genen beziehungsweise Polynukleotiden versteht man die in der Population einer Art vorhandenen offenen Leserahmen (ORF), Gene oder Allele beziehungsweise deren Polynukleotide. Die Begriffe "Gen" und "ORF" (offenen Leserahmen) werden in dieser Erfindung synonym verwendet.

Der Begriff "Polynukleotid" bezieht sich im allgemeinen auf Polyribonukleotide und Polydeoxyribonukleotide, wobei es sich um nicht modifizierte RNA oder DNA oder modifizierte RNA oder DNA handeln kann.

Der Begriff "Polypeptid" bezeichnet Peptide oder Proteine, die zwei oder mehr über Peptidbindungen verbundene Aminosäuren enthalten. Die Begriffe Polypeptid und Protein werden als Synonyme verwendet. Proteine gehören zu den Grundbausteinen aller Zellen. Sie verleihen der Zelle nicht nur Struktur, sondern sind die molekularen "Maschinen", die Stoffe transportieren, chemische Reaktionen katalysieren und Signalstoffe erkennen.

Unter "proteinogenen Aminosäuren" versteht man die Aminosäuren, die in natürlichen Proteinen, das heißt in Proteinen von Mikroorganismen, Pflanzen, Tieren und Menschen vorkommen. Hierzu gehören insbesondere L-Aminosäuren, ausgewählt aus der Gruppe L-Asparaginsäure, L-Asparagin, L-Threonin, L-Serin, L-Glutaminsäure, L-Glutamin, Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Prolin und L-Arginin, sowie Selenocystein. Dabei handelt es sich bei den proteinogenen Aminosäuren stets um α-Aminosäuren. Bis auf die Aminosäure Glycin ist für alle proteinogenen Aminosäuren das α-Kohlenstoffatom asymmentrisch (die Moleküle sind chiral): Es existieren von jeder dieser Aminosäuren zwei Enantiomere. Dabei ist nur eines der beiden Enatiomere proteinogen und zwar die L-Aminosäure: der zum Aufbau der Proteine notwendige Apparat - das Ribosom, die tRNA, die Aminoacyl-tRNA Synthetase (diese belädt die tRNA mit Aminosäuren) und andere- sind selbst auch chiral und können nur die L-Variante erkennen.

Der Begriff "Genexpression" (kurz "Expression") bezeichnet im Allgemeinen die Ausprägung der genetischen Information zu einem Phänotyp. Im engeren Sinne bezeichnet Genexpression die Transkription eines Gens zu einer RNA, die anschließende Translation der RNA zu einem Polypeptid, welches eine enzymatische Aktivität besitzen kann.

Unter dem Begriff "Überexpression" versteht man allgemein eine Erhöhung der intrazellulären Konzentration oder Aktivität einer Ribonukleinsäure, eines Proteins oder eines Enzyms im Vergleich zum Ausgangsstamm (Elternstamm) oder Wildtypstamm. Im Falle der vorliegenden Erfindung werden Thiosulfat-Sulfurtransferase-Gene beziehungsweise Polynukleotide überexprimiert, die für ein Thiosulfat-Sulfurtransferase-Polypeptid kodieren.

Unter einem "Ausgangsstamm" (Elternstamm) versteht man den Mikroorganismenstamm, an dem Maßnahmen zur Erhöhung der Produktivität einer oder mehrerer Aminosäuren, Peptide oder Proteine, bzw. Maßnahmen zur Erhöhung der Aktivität eines oder mehrerer Enzyme (z.B. eine zur Überexpression führende Maßnahme) durchgeführt werden. Bei einem Ausgangsstamm kann es sich um einen wildtypischen Stamm, aber auch um einen bereits vorher veränderten Stamm handeln (beispielsweise um einen Produktionsstamm).

Unter einem "Wildtyp" einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind.

Die im Sinne dieser Erfindung erhaltenen Mutanten zeigen eine im Vergleich zum eingesetzten Ausgangsstamm beziehungsweise Elternstamm erhöhte Ausscheidung beziehungsweise Produktion der gewünschten Aminosäure in einem Fermentationsprozess. Dabei werden die Aminosäuren in das sie umgebende Medium abgegeben oder im Zellinneren angehäuft (Akkumulation).

Der Begriff "Verstärkung" oder "erhöhte Aktivität" beschreibt in diesem Zusammenhang die Erhöhung der intrazellulären enzymatischen Aktivität eines oder mehrerer Enzyme in einem Mikroorganismus, die durch die entsprechende DNA kodiert werden.

Grundsätzlich lässt sich eine Steigerung der enzymatischen Aktivität beispielsweise dadurch erzielen, dass man die Kopienzahl der Gensequenz bzw. der Gensequenzen erhöht, welche für das Enzym kodieren, einen starken Promotor verwendet oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder einer Kombination dieser Methoden mit einem Vektor erzeugt, der das gewünschte Gen, ein Allel dieses Gens oder Teile davon und einen die Expression des Gens ermöglichenden Vektor enthält. Die heterologe Expression wird insbesondere durch Integration des Gens oder der Allele in das Chromosom der Zelle oder einem extrachromosomal replizierenden Vektor erzielt.

Einen Überblick über die Möglichkeiten zur Erhöhung der enzymatischen Aktivität in Zellen am Beispiel der Pyruvat-Carboxylase gibt DE-A-100 31 999, die hiermit als Referenz eingeführt wird und deren Offenbarungsgehalt hinsichtlich der Möglichkeiten zur Erhöhung der enzymatischen Aktivität in Zellen einen Teil der Offenbarung der vorliegenden Erfindung bildet.

Die Erhöhung der enzymatischen Aktivität lässt sich beispielsweise dadurch erzielen, dass man die Kopienzahl der entsprechenden Polynukleotide chromosomal oder extrachromosomal um mindestens eine Kopie erhöht.

Eine weit verbreitete Methode zur Erhöhung der Kopienzahl besteht darin, dass man das entsprechende Polynukleotid in einen Vektor, bevorzugt ein Plasmid, einbaut, der von einem Bakterium repliziert wird.

Geeignete Plasmidvektoren für Enterobacteriaceae sind z.B. von pACYC184 abgeleitete Kloniervektoren (Bartolome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)). Besonders geeignet sind zudem von pCL1920 (Lerner, C.G. and Inouye, M., Nucl. Acids Res. (1990) 18:4631 [PMID: 2201955]) abgeleitete Plasmide. Von "Bacterial Artificial Chromosomes" (BAC) abgeleitete Plasmidvektoren, wie z.B. pCC1BAC (EPICENTRE Biotechnologies, Madison, U.S.A.) sind ebenfalls geeignet, die Kopienzahl der entsprechenden Polynukleotide in *E. coli* zu erhöhen.

Geeignete Plasmidvektoren für *C. glutamicum* sind beispielsweise pZ1 (Menkel et al., Applied and Environmental Microbiology 64: 549-554 (1989)), pEKEx1 (Eikmanns et al., Gene 107: 69-74 (1991)) oder pHS2-1 (Sonnen et al. , Gene 107: 69-74 (1991)). Sie beruhen auf den kryptischen Plasmiden pHM1519, pBL1 oder pGA1. Andere Plasmidvektoren, wie zum Beispiel solche, die auf pCG4 (US 4,489,160) oder pNG2 (Serwold-Davis et al., FEMS Microbiology Letters 66: 119-124 (1990)) oder pAG1 (US 5,158,891) beruhen, können in gleicher Weise eingesetzt werden. Ein Übersichtsartikel zum Thema Plasmide in *C*. *glutamicum* findet sich bei Tauch et al. (Journal of Biotechnology 104, 27-40 (2003)).

Weiterhin kann man als Vektoren Transposons, Insertionselemente (IS-Elemente) oder Phagen einsetzen. Derartige genetische Systeme sind beispielsweise in den Patentschriften US 4,822,738, US 5,804,414 und US 5,804414 beschrieben. In gleicher Weise kann das in der WO 92/02627 beschriebene IS-Element ISaBl oder das Transposon Tn 45 des Plasmids pXZ10142 (zitiert im "Handbook of Corynebacterium glutamicum" (Herausgeber: L. Eggeling und M. Bott)) verwendet werden.

Eine andere verbreitete Methode zur Erzielung einer Überexpression ist das Verfahren der chromosomalen Genamplifikation. Bei dieser Methode wird mindestens eine zusätzliche Kopie des interessierenden Polynukleotids in das Chromosom eines Bakteriums eingefügt. Derartige Amplifikationsverfahren sind beispielsweise in der WO 03/014330 oder WO 03/040373 beschrieben.

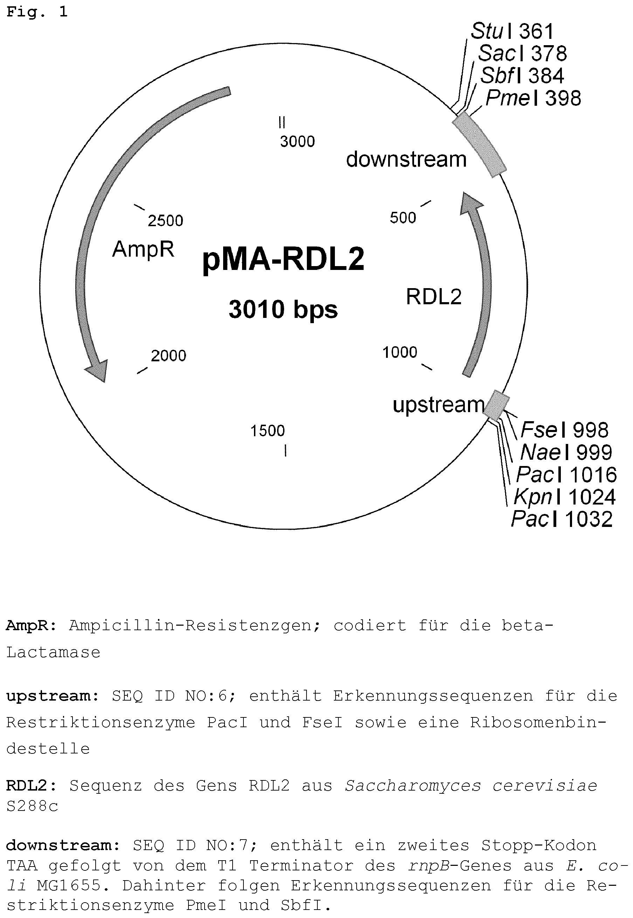

Eine weitere Methode zur Erzielung einer Überexpression besteht darin, das entsprechende Gen beziehungsweise Allel in funktioneller Weise (operably linked) mit einem Promotor beziehungsweise einer Expressionskassette zu verknüpfen. Geeignete Promotoren für *C. glutamicum* sind beispielsweise in der Fig. 1 des Übersichtsartikel von Patek et al. (Journal of Biotechnology 104(1-3), 311-323 (2003)) beschrieben. In gleicher Weise können die von Vasicova et al (Journal of Bacteriology 181, 6188-6191 (1999)) beschriebenen Varianten des dapA-Promotors, beispielsweise der Promotor A25 eingesetzt werden. Weiterhin kann der gap-Promotor von *C*. *glutamicum* (EP 06007373) verwendet werden.

Für *E. coli* sind z.B. die von Amann et al. (Gene 69(2), 301-315 (1988)) und Amann und Brosius (Gene 40(2-3), 183-190 (1985)) beschriebenen Promotoren T3, T7, SP6, M13, lac, tac und trc bekannt, welche teilweise auch für *C*. *glutamicum* verwendet werden können. Ein derartiger Promotor kann beispielsweise stromaufwärts des betreffenden Gens, typischerweise im Abstand von ungefähr 1 - 500 Nukleobasen vom Startkodon eingefügt werden. In der US 5,939,307 wird beschrieben, dass durch Einbau von Expressionskassetten oder Promotoren wie beispielsweise tac-Promotor, trp-Promotor, lpp-Promotor oder PL-Promotor und PR-Promotor des Phagen λ beispielsweise stromaufwärts des chromosomalen Threoninoperons eine Erhöhung der Expression erzielt werden konnte. In gleicher Weise können die Promotoren des Phagen T7, die gear-box-Promotoren oder der nar-Promotor verwendet werden. Derartige Expressionskassetten oder Promotoren können auch verwendet werden um, wie in der EP 0 593 792 beschrieben, plasmidgebundene Gene zu überexprimieren. Durch Verwendung des lacIQ-Allels lässt sich wiederum die Expression plasmidgebundener Gene kontrollieren (Glascock und Weickert, Gene 223, 221-231 (1998)). Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist.

Durch die Maßnahmen der Überexpression, wird die Aktivität oder Konzentration des entsprechenden Polypeptids im allgemeinen um mindestens 10%, 25%, 50%, 75%, 100%, 150%, 200%, 300%, 400% oder 500%, maximal bis 1000% oder 2000% bezogen auf die Aktivität oder Konzentration des Polypeptids im entsprechenden Ausgangsstamm (Wildtyp oder Ausgangs-Mikroorganismus) vor der zur Überexpression führenden Maßnahme, erhöht.

Methoden zur Bestimmung der enzymatischen Aktivität von Thiosulfat-Sulfurtransferasen sind zum Beispiel bei Cheng H, Donahue JL, Battle SE, Ray WK, Larson TJ, 2008, Open Microbiol J., 2:18-28 und bei Alexander K., Volini M, 1987, J. Biol. Chem., 262: 6595-6604 beschrieben.

Die Expression der vorstehend genannten Enzyme bzw. Gene ist mit Hilfe von 1- und 2-dimensionaler Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8) : 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.

Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden gentechnisch veränderte Zellen erhalten. Besonders bevorzugte Mutanten von Enzymen sind insbesondere auch solche Enzyme, die nicht mehr oder zumindest im Vergleich zum Wildtyp-Enzym vermindert feedback-inhibierbar sind.

Wird die Erhöhung der Enzymaktivität durch Erhöhung der Expression eines Enzyms bewerkstelligt, so erhöht man beispielsweise die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Enzym-Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP-A-0 472 869, im US 4,601,893 , bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO-A-96/15246, bei Malumbres et al. (Gene 134, 15-24 (1993), in JP-A-10-229891, bei Jensen und Hammer (Biotechnology and Bioengineering 58, 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.

Weiterhin eignen sich auch solche Plasmidvektoren, mit Hilfe derer man das Verfahren der Genamplifikation durch Integration in das Chromosom anwenden kann, so wie es beispielsweise von Reinscheid et al. (Applied and Environmental Microbiology 60: 126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise *Escherichia coli*), nicht aber in *Corynebacterium glutamicum* repliziert werden kann. Als Vektoren kommen beispielsweise pSUP301 (Simon et al., Bio/Technology 1: 784-791 (1983)), pK18mob oder pK19mob (Schäfer et al., Gene 145: 69-73 (1994)), pGEM-T (Promega Corporation, Madison, Wisconsin, USA), pCR2.1-TOPO (Shuman, Journal of Biological Chemistry 269: 32678-84 (1994)), pCR-BluntII-TOPO (Invitrogen, Groningen, Niederlande), pEM1 (Schrumpf et al., Journal of Bacteriology 173: 4510-4516)) oder pBGS8 (Spratt et al., Gene 41: 337-342 (1986)) in Frage. Der Plasmidvektor, der das zu amplifizierende Gen enthält, wird anschließend durch Konjugation oder Transformation in den gewünschten Stamm von *Corynebacterium glutamicum* überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al., Applied Microbiology and Biotechnology 29: 356-362 (1988), Dunican und Shivnan, Bio/Technology 7: 1067-1070 (1989) und Tauch et al., FEMS Microbiology Letters 123: 343-347 (1994) beschrieben. Nach homologer Rekombination mittels eines "cross-over"-Ereignisses enthält der resultierende Stamm mindestens zwei Kopien des betreffenden Gens. Ein ähnliches Verfahren für *E. coli* ist beispielsweise bei Link, A.J., Phillips, D. and Church, G.M. (1997), J. Bacteriology 179: 6228-6237 beschrieben.

Zur Insertion oder Deletion von DNA im Chromosom können auch Rekombinase-Vermittelte Verfahren verwendet werden, wie sie beispilsweise von Datsenko KA, Wanner BL., 2000, Proc Natl Acad Sci U S A., 97(12):6640-5 beschrieben wurden.

Unter der vorstehend und in den nachfolgenden Ausführungen verwendeten Formulierung "eine gegenüber ihrem Wildtypstamm bzw. Ausgangsstamm gesteigerte Aktivität eines Enzyms" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms zu verstehen. Weiterhin umfasst die erfindungsgemäße Zelle, welche "eine gegenüber ihrem Wildtypstamm bzw. Ausgangsstamm gesteigerte Aktivität eines Enzyms" aufweist, insbesondere auch eine Zelle, deren Wildtyp bzw. Ausgangsstamm keine oder zumindest keine nachweisbare Aktivität dieses Enzyms aufweist und die erst nach Erhöhung der Enzymaktivität, beispielsweise durch Überexpression, eine nachweisbare Aktivität dieses Enzyms zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Expression des Enzyms induziert wird.

Die durch die Maßnahmen der Erfindung erhaltenen isolierten Bakterien zeigen eine im Vergleich zum eingesetzten Ausgangsstamm beziehungsweise Elternstamm erhöhte Ausscheidung beziehungsweise Produktion der gewünschten Aminosäure in einem Fermentationsprozess.

Unter isolierten Bakterien sind die erfindungsgemäßen, isolierten beziehungsweise erzeugten Mutanten und rekombinanten Bakterien, insbesondere der Familie Enterobacteriaceae oder Corynebacteriaceae, zu verstehen, welche gegenüber dem Ausgangsstamm eine erhöhte Aktivität einer Thiosulfat-Sulfurtransferase aufweisen.

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der ProduktAusbeute (gebildetes Produkt pro verbrauchter KohlenstoffQuelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den Ausgangstamm bzw. Elternstamm bzw. den Fermentationsprozess unter Verwendung derselben verbessert.

Im erfindungsgemäßen Verfahren können die Bakterien kontinuierlich - wie beispielsweise in der PCT/EP2004/008882 beschrieben- oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Laktose, Fructose, Maltose, Melasse, Saccharose-haltige Lösungen aus der Zuckerrüben- oder Zuckerrohrherstellung, Stärke, Stärkehydrolysat und Cellulose, Öle und Fette, wie zum Beispiel Sojaöl, Sonnenblumenöl, Erdnussöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden.

Als Schwefelquelle wird gemäß der Erfindung ein Salz der Thioschwefelsäure (Thiosulfat) eingesetzt, ggf. gemeinsam mit anderen Schwefelquellen wie zum Beispiel Sulfat, Sulfit oder Dithionit.

Das Kulturmedium muss weiterhin Salze beispielsweise in Form von Chloriden, von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden. Dem Kulturmedium können überdies geeignete Vorstufen der jeweiligen Aminosäure zugesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 9,0 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete selektiv wirkende Stoffe, wie zum Beispiel Antibiotika hinzugefügt werden. Um aerobe Bedingungen aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Druck von 0,03 bis 0,2 MPa, gefahren. Die Temperatur der Kultur liegt normalerweise bei 20°C bis 45°C und vorzugsweise bei 25°C bis 40°C. Bei batch-Verfahren wird die Kultivierung solange fortgesetzt, bis sich ein Maximum der gewünschten Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Geeignete Fermentationsmedien sind unter anderem in der US 6,221,636, in der US 5,840,551, in der US 5,770,409, in der US 5, 605, 818, in der US 5, 275, 940 und in der US 4,224,409 beschrieben.

Methoden zur Bestimmung von L-Aminosäuren sind aus dem Stand der Technik bekannt. Die Analyse kann zum Beispiel so wie bei Spackman et al. (Analytical Chemistry, 30, (1958), 1190) beschrieben durch Ionenaustausch-Chromatographie mit anschließender Ninhydrin-Derivatisierung erfolgen, oder sie kann durch reversed phase HPLC erfolgen, so wie bei Lindroth et al. (Analytical Chemistry (1979) 51: 1167-1174) beschrieben.

Die auf diese Weise hergestellte Fermentationsbrühe wird anschließend zu einem festen oder flüssigen Produkt weiterverarbeitet.

Unter einer Fermentationsbrühe versteht man ein Fermentationsmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Vermehrung des Mikroorganismus und eine Bildung der gewünschten Aminosäure sicherstellt.

Bei Abschluss der Fermentation enthält die entstandene Fermentationsbrühe dementsprechend a) die infolge der Vermehrung der Zellen des Mikroorganismus entstandene Biomasse des Mikroorganismus, b) die im Laufe der Fermentation gebildete gewünschte Aminosäure, c) die im Laufe der Fermentation gebildeten organischen Nebenprodukte und d) die durch die Fermentation nicht verbrauchten Bestandteile des/der eingesetzten Fermentationsmediums/ Fermentationsmedien beziehungsweise der Einsatzstoffe wie beispielsweise Vitamine wie Biotin, Aminosäuren wie Homoserin oder Salze wie Magnesiumsulfat.

Zu den organischen Nebenprodukte gehören Stoffe, die von den bei der Fermentation eingesetzten Mikroorganismen gegebenenfalls neben der jeweiligen erwünschten L-Aminosäure erzeugt und gegebenenfalls ausgeschieden werden. Hierzu zählen L-Aminosäuren, die im Vergleich zur erwünschten Aminosäure weniger als 30%, 20% oder 10% ausmachen. Hierzu gehören weiterhin organische Säuren, die ein bis drei Carboxyl-Gruppen tragen wie zum Beispiel Essigsäure, Milchsäure, Zitronensäure, Apfelsäure oder Fumarsäure. Schließlich gehören dazu auch Zucker wie zum Beispiel Trehalose.

Typische für industrielle Zwecke geeignete Fermentationsbrühen haben einen Aminosäuregehalt von 40 g/kg bis 180 g/kg oder 50 g/kg bis 150 g/kg. Der Gehalt an Biomasse (als getrocknete Biomasse) beträgt im Allgemeinen 20 bis 50 g/kg.

### Beispiele:

### Beispiel 1: Synthese und Klonierung des Gens RDL2

Für die Expression des ORFs RDL2 (SEQ ID NO:1) aus *S. cereviseae* wurde eine Nukleotidsequenz (GENEART AG; Regensburg, Deutschland) *de novo* synthetisiert, welche aus einer *upstream*-Sequenz ("upstream", SEQ ID NO:6), der Sequenz von ORF RDL2 ("RDL2", SEQ ID NO:1) sowie aus einer *downstream-*Sequenz ("downstream", SEQ ID NO:7) besteht. Die *upstream-*Sequenz enthält Erkennungssequenzen für die Restriktionsenzyme PacI und FseI sowie eine Ribosomenbindestelle. Die *downstream-*Sequenz enthält ein zweites Stopp-Kodon TAA gefolgt von dem T1 Terminator des rnpB-Genes aus *E. coli* MG1655. Dahinter folgen Erkennungssequenzen für die Restriktionsenzyme PmeI und SbfI. Nach der Synthese wurden die Nukleotidsequenz mit SacI und KpnI geschnitten und in das ebenfalls mit SacI und KpnI geschnittene Plasmid pMA (ampR) kloniert. Das resultierende Plasmid wurde mit "pMA-RDL2" (SEQ ID NO:8, Figur 1) bezeichnet.

Ausgehend von der Aminosäuresequenz des durch den ORF RDL2 kodierten Proteins Rdl2p (Sequenz "RDL2p", SEQ ID NO:2) wurden drei verschiedene DNA-Sequenzen generiert, welche für Rdl2p-Proteine mit wildtypischer Aminosäuresequenz kodieren. Die Sequenzen wurden mit "RDL2a" (SEQ ID NO:3), "RDL2b" (SEQ ID NO:4) und "RDL2c" (SEQ ID NO:5) bezeichnet. Diese drei Sequenzen wurden jeweils zusammen mit den oben beschriebenen *upstream* und *downstream*-Sequenzen *de novo* synthetisiert und wie oben beschrieben in das Plasmid pMA kloniert(GENEART AG; Regensburg, Deutschland). Die resultierenden Plasmide wurden mit pMA-RDL2a, pMA-RDL2b und pMA-RDL2c bezeichnet. Die genannten Varianten unterscheiden sich in der Optimierung der Kodon-Verwendung (codon usage) des für das Verfahren verwendeten Mikroorganismus. So sind die Werte für die Anpassung an die Kodon-Verwendung wie folgt:
RDL2: Kodon-Verwendung nicht angepasst ("codon adaptation index" CAI=0,27)
RDL2a: Kodon-Verwendung leicht an *E. coli* angepasst (CAI=0,38)
RDL2b: Kodon-Verwendung stärker an *E. coli* angepasst (CAI=0,72)
RDL2c: Kodon-Verwendung vollständig an *E. coli* angepasst (CAI=1)

### Beispiel 2: Klonierung der ORFs RDL2, RDL2a, RDL2b sowie RDL2c in das Plasmid pME101-thrA*1-cysE-Pgap-metA*11.

Für die Expression in *E. coli* wurden die vier für das RDL2-Protein kodierenden Genvarianten jeweils in das *E. coli* Produktionsplasmid pME101-*thrA**1*-cysE-*P*gap-metA**11 (WO2007/ 077041) downstream von *metA**11 kloniert.

Dazu wurden die Gene jeweils mit den Primern RDL2-t4-f (SEQ ID NO:11) und RDL2-r (SEQ ID NO:12) unter Anwendung der Polymerase-Kettenreaktion (PCR) mit der Phusion DNA-Polymerase (Finnzymes Oy, Espoo, Finland) amplifiziert. Als Templates dienten die Plasmide pMA-RDL2, pMA-RDL2a, pMA-RDL2b und pMA-RDL2c aus Beispiel 1.
RDL2-t4-f (SEQ ID NO:11):
   5' aggacagtcgacggtaccgcaagcttcggcttcgcACTGGAAAGCGGGCAGTGAG 3'
RDL2-r (SEQ ID NO:12):
   5' AGCGCGACGTAATACGACTC 3'

Die 804 bp großen PCR-Produkte und das Plasmid pME101-*thrA*1-cysE-*P*gapA-metA**11 wurden mit den Restriktionsenzymen *SalI* und *SbfI* gespalten, ligiert und in den *E. coli* Stamm DH5α transformiert. Plasmidtragende Zellen wurden durch die Kultivierung auf LB-Agar mit 50 µg/ml Streptomycin selektioniert. Die erfolgreiche Klonierung wurde nach der Plasmid-DNA-Isolierung durch die Kontrollspaltung mit dem Restriktionsenzym AgeI nachgewiesen. Abschließend wurden die klonierten DNA-Fragmente mit den folgenden Primern sequenziert:
pME-RDL2-Seqf (SEQ ID NO:13):
   5' ATGTGGAAGCCGGACTAGAC 3'
pME-RDL2-Seqr (SEQ ID NO:14):
   5' TCGGATTATCCCGTGACAGG 3'

Die so entstandenen Plasmide wurden mit pME-RDL2, pME-RDL2a, pME-RDL2b und pME-RDL2c bezeichnet.

### Beispiel 3: Transformation von E.coli MG1655ΔmetJ Ptrc-metH Ptrc-metF PtrcF-cysPUWAM PtrcF-cysJIH Ptrc09-gcvTHP

Der L-Methionin produzierende *E. coli* Stamm MG1655Δ*metJ* metA*11 P*trc-metH* P*trc-metF* P*trcF-cysPUWAM* P*trcF-cysJIH* (im Folgenden mit DM2219 bezeichnet) wird in der Patentschrift WO 2009/043803 A2 beschrieben.

Der Stamm wurde jeweils mit den Plasmiden pME-RDL2, pME-RDL2a, pME-RDL2b und pME-RDL2c sowie mit dem Plasmid pME101-*thrA**1*-cysE-*P*gapA-metA**11 (WO2009/043803 A2) transformiert und plasmidtragende Zellen auf LB-Agar mit 50 µg/ml Streptomycin selektioniert. Die Transformanden wurden in je 10ml LB-Flüssigmedium mit 1% Glucose und 50 µg/ml Streptomycin überimpft und 16 Stunden bei 37°C kultiviert. Anschließend wurde Glycerin auf eine Enkonzentration von 10% zugesetzt und die Kulturen bei -70°C eingefroren.

### Beispiel 4: Bewertung der E.coli L-Methionin Produktions-stämme

Die Leistungsfähigkeit der *E.coli* L-Methionin-Produktionsstämme wurde durch Produktionsversuche in 100 ml Erlenmeyer-Kolben bewertet. Als Vorkulturen wurden jeweils 10 ml Vorkulturmedium (10% LB-Medium mit 2,5 g/l Glucose und 90% Minimalmedium PC1) mit 100µl Zellkultur beimpft und 10 Stunden bei 37°C kultiviert. Hiermit wurden anschließend je 10 ml PC1-Minimalmedium (Tabelle 1) auf eine OD 600 nm von 0,2 (Eppendorf Bio-Photometer; Eppendorf AG, Hamburg, Deutschland) beimpft und für 24 Stunden bei 37°C kultiviert. Die extrazelluläre L-Methionin-Konzentration wurde mit einem Aminosäureanalysator (Sykam GmbH, Eresing, Deutschland) durch Ionenaustauschchromatographie und Nachsäulenderivatisierung mit Ninhydrindetektion bestimmt. Die extrazelluläre Glucose-Konzentration wurde mit einem YSI 2700 Select Glucose Analyzer (YSI Life Sciences, Yellow Springs, Ohio, USA) bestimmt. Die Daten zeigen, dass die Expression von ORF RDL2, RDL2a, RDL2b oder RDL2c die L-Methioninkonzentration deutlich erhöht (Tabelle 2).

**Tabelle 1: Minimal Medium PC1**

| **Substanz** | **Konzentration** |
|---|---|
| ZnSO₄ * 7 H₂O | 4 mg/l |
| CuCl₂ * 2 H₂O | 2 mg/l |
| MnSO₄ * H₂O | 20 mg/l |
| CoCl₂ * 6 H₂O | 8 mg/l |
| H₃BO₃ | 1 mg/l |
| Na₂MoO₄ * 2 H₂O | 0,4 mg/l |
| MgSO₄ * 7 H₂O | 1 g/l |
| Citronensäure * 1 H₂O | 6,56 g/l |
| CaCl₂ * 2 H₂O | 40 mg/l |
| K₂HPO₄ | 8,02 g/l |
| Na₂HPO₄ | 2 g/l |
| (NH₄)₂HPO₄ | 8 g/l |
| NH₄Cl | 0,13 g/l |
| (NH₄)₂SO₃ | 5,6 g/l |
| MOPS | 5 g/l |
| NaOH 10M | auf pH 6,8 eingestellt |
| FeSO₄ * 7 H₂O | 40 mg/l |
| Thiaminhydrochlorid | 10 mg/l |
| Vitamin B12 | 10 mg/l |
| Glucose | 10 g/l |
| Isopropyl-thio-β-galaktosid (IPTG) | 2,4 mg/l |
| Spectinomycin | 50 mg/l |

**Tabelle 2: L-Methioninkonzentrationen in den Fermentationsbrühen der verschiedenen plasmidtragenden E. coli DM2219-Stämme**

| **Stamm** | **Plasmid** | **OD (600nm)** | **L-Methionin (g/l)** |
|---|---|---|---|
| DM2219 | pME101-thrA*1-cysE-PgapA-metA*11 | 7,51 | 0,43 |
| DM2219 | pME-RDL2 | 7,59 | 0,51 |
| DM2219 | pME-RDL2a | 7,72 | 0,53 |
| DM2219 | pME-RDL2b | 7,50 | 0,52 |
| DM2219 | pME-RDL2c | 7,64 | 0,54 |

### Beispiel 5: Klonierung der ORFs RDL2, RDL2a, RDL2b sowie RDL2c in das Plasmid pEC-XT99A.

Als Basisvektor zur Expression der ORFs RDL2, RDL2a, RDL2b sowie RDL2c in *C. glutamicum* wurde das *E. coli-C. glutamicum-*Shuttle-Expressionsplasmid pEC-XT99A (EP1085094B1) verwendet. Die Plasmide pMA-RDL2, pMA-RDL2a, pMA-RDL2b und pMA-RDL2c aus Beispiel 1 wurden jeweils mit den Restriktionsenzymen StuI und NaeI gespalten und in einem 0,8%igem Agarosegel aufgetrennt. Die 638 bp großen DNA-Fragmente wurden aus dem Gel ausgeschnitten und die DNA extrahiert (QIAquick Gel Extraction Kit, QIAGEN, Hilden, Deutschland). Das Expressionsplasmid pEC-XT99A wurde mit den Restriktionsenzym Ecl136II gespalten. Anschließend wurde es jeweils mit den 638 bp großen DNA-Fragmenten unter Verwendung des Ready-To-Go Ligation Kit (Amersham GE Healthcare Europe GmbH, Freiburg, Deutschland) ligiert. Der *E. coli* Stamm DH5α (Invitrogen GmbH; Darmstadt; Deutschland) wurde mit dem Ligationsansätzen transformiert und Plasmid tragende Zellen auf LB Agar mit 5 µg/ml Tetracyclin selektioniert.

Nach der Isolierung der Plasmid DNA wurde die erfolgreiche Klonierung durch eine Kontrollspaltung mit den Restriktionsenzymen PmeI und SacII nachgewiesen. Abschließend wurden die Plasmide mit den folgenden Primern sequenziert:
pECf (SEQ ID NO:9):
   5' TACTGCCGCCAGGCAAATTC 3'
pECr (SEQ ID NO:10):
   5' TTTGCGCCGACATCATAACG 3'

Die Plasmidkonstrukte, bei denen der plasmideigene trc-Promoter und die für RDL2 kodierenden ORFs gleich orientiert sind, wurden wie folgt bezeichnet: pEC-RDL2, pEC-RDL2a, pEC-RDL2b und pEC-RDL2c.

### Beispiel 6: Transformation von C. glutamicum M1179

Der Stamm *Corynebacterium glutamicum* M1179 ist ein ethioninresistenter L-Methionin Produzent (WO2007/011939). Er wurde bei der Deutschen Sammlung für Mikroorganismen und Zellkulturen (DSMZ, Braunschweig, Deutschland) am 18.5.2005 in Übereinstimmung mit dem Budapester Abkommen als DSM17322 hinterlegt.

Die Plasmide pEC-RDL2, pEC-RDL2a, pEC-RDL2b, pEC-RDL2c bzw. pEC-XT99A wurden nach Elektroporationsbedingungen von Tauch et al. (1994, FEMS Microbiological Letters, 123: 343-347) in den Stamm M1179 elektroporiert.

Die Selektion plasmidtragender Zellen erfolgte auf LB-Agar mit 5µg/ml Tetracyclin. Die Plasmid-DNA der Transformanden wurde isoliert (Peters-Wendisch et al., 1998, Microbiology 144, 915-927) und durch Restriktionsspaltung und Gelelektrophorese überprüft. Die so entstandenen Stämme wurden mit M1179/pEC-RDL2, M1179/pEC-RDL2a, M1179/pEC-RDL2b, M1179/pEC-RDL2c und M1179/pEC-XT99A bezeichnet.

### Beispiel 7: Bewertung der C. glutamicum L-Methionin-Produktionsstämme

Die hergestellten *C. glutamicum* Stämme M1179/pEC-RDL2, M1179/pEC-RDL2a, M1179/pEC-RDL2b, M1179/pEC-RDL2c und M1179/pEC-XT99A wurden in einem zur Produktion von L-Methionin geeigneten Nährmedium kultiviert und der L-Methioningehalt im Kulturüberstand bestimmt.

Dazu wurden die Stämme zunächst auf Agarplatten (Hirn-Herz Agar mit Kanamycin (25 mg/l)) ausgestrichen und 24 Stunden bei 33°C inkubiert. Von diesen Agarplattenkulturen wurden Zellen in jeweils 10 ml Medium HH (Hirn-Herz Bouillon, Merck, Darmstadt, Deutschland) mit 5 mg/l Tetracyclin überimpft und 24 Stunden in 100 ml Erlenmeyerkolben bei 33°C bei 240 rpm geschüttelt. Hiermit wurden anschließend je 10 ml PM-Medium (Tabelle 3) auf eine OD 660 nm von 0,1 (Genios, Tecan Deutschland GmbH, Crailsheim, Deutschland) beimpft und für 24 Stunden bei 33°C in 100 ml Erlenmeyerkolben mit Schikanen kultiviert.

**Tabelle 3: PM-Medium**

| **Substanz** | **Konzentration** |
|---|---|
| Glukose | 50 g/l |
| (NH₄)₂S₂O₃ | 10 g/l |
| (NH₄)Cl | 10 g/l |
| MgSO₄ * 7 H₂O | 0,4 g/l |
| KH₂PO₄ | 0,6 g/l |
| Yeast Extract (Difco) | 10 g/l |
| FeSO₄ * 7 H₂O | 2 mg/l |
| MnSO₄ * H₂O | 2 mg/l |
| Ammoniakwasser | auf pH 7,8 eingestellt |
| Thiamin * HCl | 1 mg/l |
| Vitamin B12 | 0,2 mg/l |
| Biotin | 0,1 mg/l |
| Pyridoxin * HCl (Vitamin B6) | 5 mg/l |
| Threonin | 238 mg/l |
| CaCO3 | 50 g/l |
| Tetracyclin | 5 mg/l |
| Isopropyl-thio-β-galaktosid (IPTG) | 1 mM |

Nach 24 Stunden wurde die OD bei 660nm bestimmt (Genios, Tecan Deutschland GmbH, Crailsheim, Deutschland) und die Konzentration gebildeten L-Methionins gemessen. L-Methionin wurde mit einem Aminosäureanalysator (Sykam GmbH, Eresing, Deutschland) durch Ionenaustauschchromatographie und Nachsäulenderivatisierung mit Ninhydrindetektion bestimmt. Tabelle 4 zeigt die optischen Dichten und die L-Methioninkonzentrationen der Kulturen. Die Expression von ORF RDL2, RDL2a, RDL2b oder RDL2c hat eine deutliche Steigerung der L-Methioninkonzentration zur Folge.

**Tabelle 4: Methioninkonzentrationen in den Fermentationsbrühen der verschiedenen plasmidtragenden C. glutamicum M1179-Stämme**

| **Stamm** | **OD (600nm)** | **L-Methionin (g/l)** |
|---|---|---|
| M1179/pEC-XT99A | 25,7 | 0,47 |
| M1179/pEC-RDL2 | 25,8 | 0,50 |
| M1179/pEC-RDL2a | 24,1 | 0,50 |
| M1179/pEC-RDL2b | 26,2 | 0,55 |
| M1179/pEC-RDL2c | 24,5 | 0,54 |

## Patentansprüche

1. Ein Verfahren zur fermentativen Herstellung schwefelhaltiger Aminosäuren ausgewählt aus der Gruppe L-Methionin, L-Cystein, L-Cystin, L-Homocystein und L-Homocystin, enthaltend die Schritte:
a) Bereitstellen eines Mikroorganismus der Familie Enterobacteriaceae oder eines Mikroorganismus der Familie Corynebacteriaceae, der ein Gen kodierend für ein Polypeptid mit der Aktivität einer Thiosulfat-Sulfurtransferase überexprimiert;
b) Fermentieren des Mikroorganismus aus a) in einem Medium, welches eine anorganische Schwefelquelle aus der Gruppe Salz der Thioschwefelsäure oder eine Mischung aus einem Salz der Thioschwefelsäure und einem Salz der Schwefelsäure enthält, wobei eine Fermentationsbrühe erhalten wird, und
c) Anreichern der schwefelhaltigen Aminosäure in der Fermentationsbrühe aus b).

2. Das Verfahren gemäß Anspruch 1, **dadurch gekennzeichnet, dass** der Mikroorganismus ein oder mehrere Gene kodierend für ein Polypeptid mit der Aktivität einer Thiosulfat-Sulfurtransferase überexprimiert, wobei das Polypeptid mit der Aktivität einer Thiosulfat-Sulfurtransferase ausgewählt ist aus den folgenden a) bis d) :
a) ein Polypeptid bestehend aus oder enthaltend die Polypeptide Rdl2p, GlpE, PspE, YgaP, ThiI, YbbB, SseA, YnjE, YceA, YibN, NCgl0671, NCgl1369, NCgl2616, NCgl0053, NCgl0054, NCgl2678, NCgl2890; eine Thiosulfat-Sulfurtransferase aus Säugetieren, beispielsweise die Thiosulfat-Sulfurtransferase aus der Rinderleber (*Bos taurus*); bevorzugt Rdl2p, GlpE, PspE und besonders bevorzugt Rdl2p;
b) ein Polypeptid bestehend aus oder enthaltend die in SEQ ID NO: 2 gezeigte Aminosäuresequenz;
c) ein Polypeptid mit einer Aminosäuresequenz, die zu 70% oder mehr identisch ist mit der Aminosäuresequenz von a) oder b), wobei das Polypeptid Thiosulfat-Sulfurtransferase-Aktivität aufweist;
d) ein Polypeptid, das eine Aminosäuresequenz enthaltend eine Deletion, Substitution, Insertion und/oder Addition von 1 bis 45 Aminosäureresten bezüglich der in SEQ ID NO: 2 gezeigten Aminosäuresequenz aufweist, wobei das Polypeptid Thiosulfat-Sulfurtransferase-Aktivität aufweist.

3. Das Verfahren nach einem der Ansprüche 1 bis 2, **dadurch gekennzeichnet**, das die Expression des Gens kodierend für ein Polypeptid mit der Aktivität einer Thiosulfat-Sulfurtransferase durch eine oder mehrere Maßnahmen ausgewählt aus der folgenden Gruppe erhöht wird:
a) die Expression des Gens steht unter der Kontrolle eines Promotors, der in dem für das Verfahren verwendeten Mikroorganismus zu einer gegenüber dem Ausgangsstamm verstärkten Expression führt;
b) Erhöhung der Kopienzahl des Gens kodierend für ein Polypeptid mit der Aktivität einer Thiosulfat-Sulfurtransferase gegenüber dem Ausgangsstamm; vorzugsweise durch Einfügen des Gens in Plasmide oder durch Integration des Gens in das Chromosom des Mikroorganismus in mehreren Kopien;
c) die Expression des Gens erfolgt unter Verwendung einer Ribosomen-Bindestelle, die in dem für das Verfahren verwendeten Mikroorganismus zu einer gegenüber dem Ausgangsstamm verstärkten Translation führt;
d) die Expression des Gens wird durch eine Optimierung der Kodon-Verwendung (codon usage) bezogen auf den für das Verfahren verwendeten Mikroorganismus verstärkt;
e) die Expression des Gens wird durch die Reduzierung von mRNA-Sekundärstrukturen in der vom Gen transkribierten mRNA verstärkt;
f) die Expression des Gens wird durch die Eliminierung von RNA-Polymerase-Terminatoren in der vom Gen transkribierten mRNA verstärkt;
g) die Expression des Gens erfolgt unter Verwendung mRNA-stabilisierender Sequenzen in der vom Gen transkribierten mRNA.

4. Das Verfahren nach einem der Ansprüche 1 bis 3, **dadurch gekennzeichnet, dass** es sich bei dem Salz der Thioschwefelsäure um ein Salz ausgewählt aus der Gruppe Alkalisalz, Erdalkalisalz, Ammoniumsalz und Mischungen davon, bevorzugt Ammoniumsalz, handelt.

5. Das Verfahren nach einem der Ansprüche 1 bis 3, **dadurch gekennzeichnet, dass** es sich bei dem Salz der Schwefelsäure um ein Salz ausgewählt aus der Gruppe Alkalisalz, Erdalkalisalz, Ammoniumsalz und Mischungen davon, bevorzugt Ammoniumsalz, handelt.

6. Das Verfahren nach einem der Ansprüche 1 bis 5, **dadurch gekennzeichnet, dass** während der Fermentation der Anteil des Salzes der Thioschwefelsäure bezogen auf den Gesamtgehalt an anorganischem Schwefel im Medium und in der Fermentationsbrühe auf mindestens 5 mol% gehalten wird.

7. Das Verfahren gemäß irgend einem der Ansprüche 1 bis 6, **dadurch gekennzeichnet, dass** es sich bei der schwefelhaltigen Aminosäure um L-Methionin handelt.

8. Das Verfahren gemäß irgend einem der Ansprüche 1 bis 7, **dadurch gekennzeichnet, dass** es sich bei dem Mikroorganismus um die Gattung *Escherichia,* bevorzugt die Art *Escherichia coli,* handelt.

9. Das Verfahren gemäß irgend einem der Ansprüche 1 bis 7, **dadurch gekennzeichnet, dass** es sich bei dem Mikroorganismus um die Gattung *Corynebacterium,* bevorzugt die Art *Corynebacterium glutamicum,* handelt.

10. Das Verfahren gemäß irgend einem der Ansprüche 1 bis 9, **dadurch gekennzeichnet, dass** der Mikroorganismus ausgewählt ist aus
- *Corynebacterium glutamicum* mit erhöhter Aktivität und/oder Expression der Aspartatkinase und Abschwächung oder Deletion des Regulatorproteins McbR im Vergleich zum Ausgangsstamm;
- *Escherichia coli,* mit erhöhter Aktivität und/oder Expression der Aspartatkinase und Abschwächung oder Deletion des Regulatorproteins MetJ im Vergleich zum Ausgangsstamm.

11. Ein Mikroorganismus ausgewählt aus
- *Corynebacterium glutamicum* mit erhöhter Aktivität und/oder Expression der Aspartatkinase und Abschwächung oder Deletion des Regulatorproteins McbR im Vergleich zum Ausgangsstamm;
- *Escherichia coli,* mit erhöhter Aktivität und/oder Expression der Aspartatkinase und Abschwächung oder Deletion des Regulatorproteins MetJ im Vergleich zum Ausgangsstamm;
wobei der Mikroorganismus L-Methionin ausscheidet oder produziert, **dadurch gekennzeichnet, dass** der Mikroorganismus ein Gen kodierend für ein Polypeptid mit der Aktivität einer Thiosulfat-Sulfurtransferase überexprimiert.

12. Der Mikroorganismus nach Anspruch 11, **dadurch gekennzeichnet, dass** dieser Mikroorganismus ein Gen kodierend für ein Polypeptid mit der Aktivität einer Thiosulfat-Sulfurtransferase überexprimiert, wobei das Polypeptid mit der Aktivität einer Thiosulfat-Sulfurtransferase so wie in Anspruch 2 definiert ist.

13. Der Mikroorganismus nach einem der Ansprüche 11 bis 12, **dadurch gekennzeichnet, dass** sich der Ausgangsstamm ableitet von einem Mikroorganismus aus der Gruppe bestehend aus *Escherichia coli* MG1655, *Escherichia coli* W3110, *Escherichia coli* DH5α, *Escherichia coli* DH10B, *Escherichia coli* BW2952, *Escherichia coli* REL606, *Corynebacterium glutamicum* ATCC13032, *Corynebacterium glutamicum* R, *Corynebacterium glutamicum* DSM20411 (alte Bezeichnung *Brevibacterium flavum*), *Corynebacterium glutamicum* DSM20412 (alte Bezeichnung *Brevibacterium lactofermentum*), *Corynebacterium glutamicum* DSM1412 (alte Bezeichnung *Brevibacterium lactofermentum), Corynebacterium efficiens* YS-314^{T} (=DSM44549), *Corynebacterium glutamicum* ATCC21608, *Corynebacterium glutamicum* DSM17322.

## Claims

1. Process for the fermentative preparation of sulphur-containing amino acids chosen from the group of L-methionine, L-cysteine, L-cystine, L-homocysteine and L-homocystine, comprising the steps:
a) provision of a microorganism of the family Enterobacteriaceae or of a microorganism of the family Corynebacteriaceae which overexpresses a gene coding for a polypeptide with the activity of a thiosulphate sulphurtransferase;
b) fermentation of the microorganism from a) in a medium which contains an inorganic source of sulphur from the group of salt of thiosulphuric acid or a mixture of a salt of thiosulphuric acid and a salt of sulphuric acid, a fermentation broth being obtained, and
c) concentration of the sulphur-containing amino acid in the fermentation broth from b).

2. Process according to Claim 1, **characterized in that** the microorganism overexpresses one or more genes coding for a polypeptide with the activity of a thiosulphate sulphurtransferase, the polypeptide with the activity of a thiosulphate sulphurtransferase being chosen from the following a) to d):
a) a polypeptide consisting of or containing the polypeptides Rdl2p, GlpE, PspE, YgaP, ThiI, YbbB, SseA, YnjE, YceA, YibN, NCgl0671, NCgl1369, NCgl2616, Nagl0053, Nagl0054, Nagl2678, NCgl2890; a thiosulphate sulphurtransferase from mammals, for example the thiosulphate sulphurtransferase from the bovine liver (*Bos taurus*); preferably Rdl2p, GlpE, PspE and particularly preferably Rdl2p;
b) a polypeptide consisting of or containing the amino acid sequence shown in SEQ ID NO: 2;
c) a polypeptide with an amino acid sequence which is identical to the extent of 70 % or more to the amino acid sequence of a) or b), the polypeptide having thiosulphate sulphurtransferase activity;
d) a polypeptide which has an amino acid sequence containing a deletion, substitution, insertion and/or addition of from 1 to 45 amino acid residues with respect to the amino acid sequence shown in SEQ ID NO: 2, the polypeptide having thiosulphate sulphurtransferase activity.

3. Process according to one of Claims 1 to 2, **characterized in that** the expression of the gene coding for a polypeptide with the activity of a thiosulphate sulphurtransferase is increased by one or more measures chosen from the following group:
a) the expression of the gene is under the control of a promoter which, in the microorganism used for the process, leads to an amplified expression compared with the starting strain;
b) increasing the number of copies of the gene coding for a polypeptide with the activity of a thiosulphate sulphurtransferase compared with the starting strain; preferably by insertion of the gene into plasmids or by integration of the gene into the chromosome of the microorganism in several copies;
c) the expression of the gene is effected using a ribosome binding site, which leads to an increased translation in the microorganism used for the process compared with the starting strain;
d) the expression of the gene is amplified by an optimization of the codon usage with respect to the microorganism used for the process;
e) the expression of the gene is amplified by reduction of mRNA secondary structures in the mRNA transcribed by the gene;
f) the expression of the gene is amplified by elimination of RNA polymerase terminators in the mRNA transcribed by the gene;
g) the expression of the gene is effected using mRNA-stabilizing sequences in the mRNA transcribed by the gene.

4. Process according to one of Claims 1 to 3, **characterized in that** the salt of thiosulphuric acid is a salt chosen from the group of alkali metal salt, alkaline earth metal salt, ammonium salt and mixtures thereof, preferably ammonium salt.

5. Process according to one of Claims 1 to 3, **characterized in that** the salt of sulphuric acid is a salt chosen from the group of alkali metal salt, alkaline earth metal salt, ammonium salt and mixtures thereof, preferably ammonium salt.

6. Process according to one of Claims 1 to 5, **characterized in that** during the fermentation the content of the salt of thiosulphuric acid, based on the total content of inorganic sulphur in the medium and in the fermentation broth, is kept at at least 5 mol%.

7. Process according to any one of Claims 1 to 6, **characterized in that** the sulphur-containing amino acid is L-methionine.

8. Process according to any one of Claims 1 to 7, **characterized in that** the microorganism is the genus *Escherichia,* preferably the species *Escherichia coli.*

9. Process according to any one of Claims 1 to 7, **characterized in that** the microorganism is the genus *Corynebacterium,* preferably the species *Corynebacterium glutamicum.*

10. Process according to any one of Claims 1 to 9, **characterized in that** the microorganism is chosen from
- *Corynebacterium glutamicum* with increased activity and/or expression of aspartate kinase and attenuation or deletion of the regulator protein McbR compared with the starting strain;
- *Escherichia coli* with increased activity and/or expression of aspartate kinase and attenuation or deletion of the regulator protein MetJ compared with the starting strain.

11. Microorganism chosen from
- *Corynebacterium glutamicum* with increased activity and/or expression of aspartate kinase and attenuation or deletion of the regulator protein McbR compared with the starting strain;
- *Escherichia coli* with increased activity and/or expression of aspartate kinase and attenuation or deletion of the regulator protein MetJ compared with the starting strain;
wherein the microorganism secretes or produces L-methionine, **characterized in that** the microorganism overexpresses a gene coding for a polypeptide with the activity of a thiosulphate sulphurtransferase.

12. Microorganism according to Claim 11, **characterized in that** this microorganism overexpresses a gene coding for a polypeptide with the activity of a thiosulphate sulphurtransferase, the polypeptide with the activity of a thiosulphate sulphurtransferase being as defined in Claim 2.

13. Microorganism according to any one of Claims 11 to 12, **characterized in that t**he starting strain is derived from a microorganism from the group consisting of *Escherichia coli* MG1655, *Escherichia coli* W3110, *Escherichia coli* DH5α, *Escherichia coli* DH10B, *Escherichia coli* BW2952, *Escherichia coli* REL606, *Corynebacterium glutamicum* ATCC13032, *Corynebacterium glutamicum* R, *Corynebacterium glutamicum* DSM20411 (former name *Brevibacterium flavum*), *Corynebacterium glutamicum* DSM20412 (former name *Brevibacterium lactofermentum*), *Corynebacterium glutamicum* DSM1412 (former name *Brevibacterium lactofermentum*), *Corynebacterium efficiens* YS-314^{T} (= DSM44549), *Corynebacterium glutamicum* ATCC21608, *Corynebacterium glutamicum* DSM17322.

## Revendications

1. Procédé pour la production par fermentation d'acides aminés contenant du soufre, choisis dans le groupe formé par la L-méthionine, la L-cystéine, la L-cystine, la L-homocystéine et la L-homocystine, contenant les étapes :
a) mettre à disposition un micro-organisme de la famille des Enterobacteriaceae ou un micro-organisme de la famille des Corynebacteriaceae, qui surexprime un gène codant pour un polypeptide présentant l'activité d'une thiosulfate-soufre transférase ;
b) faire fermenter le micro-organisme de a) dans un milieu qui contient une source de soufre inorganique du groupe formé par un sel de l'acide thiosulfurique ou un mélange d'un sel de l'acide thiosulfurique et d'un sel de l'acide sulfurique, un bouillon de fermentation étant obtenu et
c) enrichir l'acide aminé contenant du soufre dans le bouillon de fermentation de b).

2. Procédé selon la revendication 1, **caractérisé en ce que** le micro-organisme surexprime un ou plusieurs gènes codant pour un polypeptide présentant l'activité d'une thiosulfate-soufre transférase, le polypeptide présentant l'activité d'une thiosulfate-soufre transférase étant choisi parmi les polypeptides a) à d) suivantes :
a) un polypeptide constitué par ou contenant les polypeptides Rdl2p, GlpE, PspE, YgaP, ThiI, YbbB, SseA, YnjE, YceA, YibN, NCgl0671, NCgl1369, NCgl2616, NCgl0053, NCgl0054, NCgl2678, NCgl2890 ; une thiosulfate-soufre transférase de mammifères, par exemple la thiosulfate-soufre transférase du foie de boeuf (*Bos taurus*) ; de préférence Rdl2p, GlpE, PspE et de manière particulièrement préférée Rdl2p ;
b) un polypeptide constitué par ou contenant la séquence d'acides aminés représentée dans la séquence SEQ ID NO : 2 ;
c) un polypeptide présentant une séquence d'acides aminés qui est identique à raison de 70% ou plus à la séquence d'acides aminés de a) ou de b), le polypeptide présentant une activité de thiosulfate-soufre transférase ;
d) un polypeptide qui présente une séquence d'acides aminés contenant une délétion, une substitution, une insertion et/ou une addition de 1 à 45 résidus d'acide aminé par rapport à la séquence d'acides aminés représentée dans la séquence SEQ ID NO : 2, le polypeptide présentant une activité de thiosulfate-soufre transférase.

3. Procédé selon l'une quelconque des revendications 1 à 2, **caractérisé en ce que** l'expression du gène codant pour un polypeptide présentant l'activité d'une thiosulfate-soufre transférase est augmentée par une ou plusieurs mesures choisies dans le groupe suivait :
a) l'expression du gène est sous le contrôle d'un promoteur qui conduit à une expression renforcée par rapport à celle de la souche de départ dans le micro-organisme utilisé pour le procédé ;
b) augmentation du nombre de copies du gène codant pour un polypeptide présentant l'activité d'une thiosulfate-soufre transférase par rapport à la souche de départ ; de préférence par introduction du gène dans des plasmides ou par intégration du gène dans le chromosome du micro-organisme en plusieurs copies ;
c) l'expression du gène a lieu avec utilisation d'un site de liaison ribosomique qui conduit, dans le micro-organisme utilisé pour le procédé, à une traduction renforcée par rapport à celle de la souche de départ ;
d) l'expression du gène est renforcée par une optimisation de l'utilisation de codons (codon usage) par rapport à celle du micro-organisme utilisé pour le procédé ;
e) l'expression du gène est renforcée par réduction de structures secondaires d'ARNm dans l'ARNm transcrit par le gène ;
f) l'expression du gène est renforcée par l'élimination de terminateurs de l'ARN polymérase dans l'ARNm transcrit par le gène ;
g) l'expression du gène a lieu avec utilisation de séquences stabilisant l'ARNm dans l'ARNm transcrit par le gène.

4. Procédé selon l'une quelconque des revendications 1 à 3, **caractérisé en ce qu'**il s'agit, pour le sel de l'acide thiosulfurique, d'un sel choisi dans le groupe formé par un sel de métal alcalin, un sel de métal alcalino-terreux, un sel d'ammonium et les mélanges de ceux-ci, de préférence d'un sel d'ammonium.

5. Procédé selon l'une quelconque des revendications 1 à 3, **caractérisé en ce qu'**il s'agit, pour le sel de l'acide sulfurique, d'un sel choisi dans le groupe formé par un sel de métal alcalin, un sel de métal alcalino-terreux, un sel d'ammonium et les mélanges de ceux-ci, de préférence d'un sel d'ammonium.

6. Procédé selon l'une quelconque des revendications 1 à 5, **caractérisé en ce que**, pendant la fermentation, la proportion du sel de l'acide thiosulfurique est maintenue à au moins 5% en mole par rapport à la teneur totale en soufre inorganique dans le milieu et dans le bouillon de fermentation.

7. Procédé selon l'une quelconque des revendications 1 à 6, **caractérisé en ce qu'**il s'agit, pour l'acide aminé contenant du soufre, de L-méthionine.

8. Procédé selon l'une quelconque des revendications 1 à 7, **caractérisé en ce qu'**il s'agit, pour le micro-organisme, du genre *Escherichia,* de préférence de l'espèce *Escherichia coli.*

9. Procédé selon l'une quelconque des revendications 1 à 7, **caractérisé en ce qu'**il s'agit, pour le micro-organisme, du genre *Corynebacterium,* de préférence de l'espèce *Corynebacterium glutamicum.*

10. Procédé selon l'une quelconque des revendications 1 à 9, **caractérisé en ce que** le micro-organisme est choisi parmi
- *Corynebacterium glutamicum* présentant une activité et/ou une expression augmentée(s) de l'aspartate kinase et un affaiblissement ou une suppression de la protéine régulatrice McbR par rapport à la souche de départ ;
- *Escherichia coli* présentant une activité et/ou une expression augmentée(s) de l'aspartate kinase et un affaiblissement ou une suppression de la protéine régulatrice MetJ par rapport à la souche de départ.

11. Micro-organisme choisi parmi
- *Corynebacterium glutamicum* présentant une activité et/ou une expression augmentée(s) de l'aspartate kinase et un affaiblissement ou une suppression de la protéine régulatrice McbR par rapport à la souche de départ ;
- *Escherichia coli* présentant une activité et/ou une expression augmentée(s) de l'aspartate kinase et un affaiblissement ou une suppression de la protéine régulatrice MetJ par rapport à la souche de départ ;
le micro-organisme excrétant ou produisant de la L-méthionine, **caractérisé en ce que** le micro-organisme surexprime un gène codant pour un polypeptide présentant l'activité d'une thiosulfate-soufre transférase.

12. Micro-organisme selon la revendication 11, **caractérisé en ce que** ce micro-organisme surexprime un gène codant pour un polypeptide présentant l'activité d'une thiosulfate-soufre transférase, le polypeptide présentant l'activité d'une thiosulfate-soufre transférase étant défini comme dans la revendication 2.

13. Micro-organisme selon l'une quelconque des revendications 11 à 12, **caractérisé en ce que** la souche de départ est dérivée d'un micro-organisme du groupe constitué par *Escherichia coli* MG1655, *Escherichia coli* W3110, *Escherichia coli* DH5α, *Escherichia coli* DH10B, *Escherichia coli* BW2952, *Escherichia coli* REL606, *Corynebacterium glutamicum* ATCC13032, *Corynebacterium glutamicum* R, *Corynebacterium glutamicum* DSM20411 (ancienne dénomination *Brevibacterium flavum*)*, Corynebacterium glutamicum* DSM20412 (ancienne dénomination *Brevibacterium lactofermentum*), *Corynebacterium glutamicum* DSM1412 (ancienne dénomination *Brevibacterium lactofermentum*), *Corynebacterium efficiens* YS-314^{T} (=DSM44549), *Corynebacterium glutamicum* ATCC21608, *Corynebacterium glutamicum* DSM17322.
